# EUROPEAN PATENT APPLICATION

(11) **EP 4 129 388 A1**
(43) Date of publication of application: **08.02.2023**
(21) Application number: 21781649.5
(22) Date of filing: 31.03.2021
(51) Int. Cl.: A61M 25/10, A61B 18/04

(54) **BALLOON CATHETER AND BALLOON CATHETER SYSTEM**

(30) Priority: 31.03.2020 JP 2020064627
(71) Applicant: Toray Industries, Inc., Tokyo 103-8666 (JP)
(72) Inventor: TSUKAMOTO, Kota, Tokyo 103-8666 (JP); TANAHASHI, Akio, Otsu-shi, Shiga 520-8558 (JP)
(74) Representative: Kador & Partner PartG mbB
(86) International application number: PCT/JP2021/013780
(87) International publication number: WO 2021/201078

(57) **Abstract**

The present invention makes it possible to highly precisely determine a surface temperature of a balloon. A balloon catheter (15) includes: a balloon (25); an outer cylinder shaft (30) connected to a proximal end (25b) of the balloon (25); an inner cylinder shaft (35) passing through the outer cylinder shaft (30) to extend into the balloon (25) to be connected to a distal end (25a) of the balloon (25); and a heating component (40) disposed in the balloon (25) for heating a liquid in the balloon (25). A liquid delivery path (LP) in communication with the balloon (25) is formed between the outer cylinder shaft and the inner cylinder shaft. A temperature sensor (45) is disposed in the liquid delivery path (LP).

## Description

### Technical Field

The present invention relates to a balloon catheter and a balloon catheter system.

### Background Art

A catheter ablation therapy is a treatment method that uses a catheter inserted into a body to ablate a target site in the body. As an example, a target site is destroyed by ablation to treat diseases such as arrhythmia due to atrial fibrillation, endometriosis, cancer, etc. As disclosed in JP3611799B and JP4747141B, a balloon catheter having a balloon at a distal end is known as a catheter used for catheter ablation therapy.

When a balloon catheter is being inserted into a body, its balloon is deflated and stretched in a longitudinal direction of the balloon catheter. Then, a liquid is supplied to the balloon catheter inserted in the body so that the balloon is inflated. Since a temperature of the liquid in the balloon is adjusted, a surface temperature of the balloon can be controlled. By bringing the balloon whose surface temperature has been adjusted to a predetermined one into contact with a circumferential target site, e.g., a connection of a vein to an atrium, the circumferential target site can be ablated at once.

In a therapy using a balloon catheter, it is important to precisely understand a surface temperature of a balloon. To this end, the balloon catheters disclosed in JP3611799B and JP4747141B are provided with a temperature sensor for measuring a surface temperature of the balloon. JP3611799B uses a temperature sensor attached to an inner surface of the balloon. However, it is not easy to stably install a temperature sensor on a surface of a balloon which inflates from a deflated state. In view of this, JP4747141B proposes that a balloon has a two-layer structure, and that a temperature sensor is located between the layers. However, such a balloon catheter is difficult to actually manufacture in terms of balloon fabrication, installation of a thermosensitive part of a temperature sensor, handling of a lead wire of the temperature sensor, etc. Thus, the balloon catheter of JP4747141B has not been widely used. Namely, it has been difficult for a conventional balloon catheter to precisely determine a surface temperature of a balloon.

### Disclosure

The present invention has been made in view of the above circumstances. An object of the present invention is to make it possible that a surface temperature of a balloon is highly precisely determined.

A first balloon catheter of the present invention comprises: a balloon; an outer cylinder shaft connected to a proximal end of the balloon; an inner cylinder shaft passing through the outer cylinder shaft to extend into the balloon to be connected to a distal end of the balloon; a heating component disposed in the balloon for heating a liquid in the balloon; and a temperature sensor disposed in a liquid delivery path formed between the outer cylinder shaft and the inner cylinder shaft to be in communication with the balloon.

In the first balloon catheter of the present invention, a length along a longitudinal direction from a distal end of the outer cylinder shaft up to the temperature sensor may be between 5 mm or more and 150 mm or less.

In the first balloon catheter of the present invention, the temperature sensor may be attached to the inner cylinder shaft; the inner cylinder shaft may relatively be movable with respect to the outer cylinder shaft; and when the inner cylinder shaft is relatively moved to a distal side with respect to the outer cylinder shaft so that the balloon is stretched, the temperature sensor may be positioned in the liquid delivery path between the outer cylinder shaft and the inner cylinder shaft.

In the first balloon catheter of the present invention, the temperature sensor may be attached to the outer cylinder shaft.

In the first balloon catheter of the present invention, the temperature sensor may include a thermosensitive part, and a lead wire connected to the thermosensitive part, and the lead wire may be fixed on the inner cylinder shaft or the outer cylinder shaft, while the thermosensitive part may be apart from the inner cylinder shaft and the outer cylinder shaft.

A second balloon catheter of the present invention comprises: a balloon; an outer cylinder shaft connected to a proximal end of the balloon; an inner cylinder shaft passing through the outer cylinder shaft to extend into the balloon to be connected to a distal end of the balloon, the inner cylinder shaft forming between it and the outer cylinder shaft a liquid delivery path in communication with the balloon; a coil electrode disposed in the balloon for heating a liquid in the balloon by applying a high-frequency current to the liquid during high-frequency voltage supply; and a temperature sensor disposed at a position shielded from the high-frequency current.

A first balloon catheter system of the present invention may comprise: one of the aforementioned first and second balloon catheters according to the present invention; and a control unit electrically connected to the temperature sensor and configured to adjust an output of the heating component based on an output of the temperature sensor.

A second balloon catheter system of the present invention may comprise: one of the aforementioned first and second balloon catheters according to the present invention; and a control unit electrically connected to the temperature sensor and configured to determine a surface temperature of the balloon based on an output of the temperature sensor.

In the first and second balloon catheter systems of the present invention, the control unit may have a display for displaying the surface temperature.

In the first and second balloon catheter systems of the present invention, the control unit may first determine a temperature fluctuation of the liquid in the liquid delivery path from an output of the temperature sensor, and then may determine the surface temperature of the balloon based on the temperature fluctuation.

In the first and second balloon catheter systems of the present invention, the control unit may first determine a temperature fluctuation of the liquid in the liquid delivery path from an output of the temperature sensor, and then may determine a local maximum value of the temperature fluctuation as the surface temperature of the balloon.

The first and second balloon catheters of the present invention may further comprise an agitator that repeats supply of the liquid to the liquid delivery path and discharge of the liquid from the liquid delivery path at a regular cycle, and the control unit may acquire an output from the temperature sensor at an interval less than the regular cycle.

A third balloon catheter system of the present invention comprises: one of the aforementioned first and second balloon catheters according to the present invention; and an agitator that repeats supply of a predetermined amount of the liquid to the liquid delivery path and discharge of the predetermined amount of the liquid from the liquid delivery path.

The first to third balloon catheter systems of the present invention may further comprise an agitator that repeats supply of a predetermined amount of the liquid to the liquid delivery path and discharge of the predetermined amount of the liquid from the liquid delivery path, wherein a length [mm] along a longitudinal direction from the distal end of the outer cylinder shaft to the temperature sensor may be a value equal to or less than a value obtained by dividing the predetermined amount [mm³] by a cross-sectional area [mm²] of the liquid delivery path.

In the first to third balloon catheter systems of the present invention, a wiring electrically connected to the heating component and the control unit may be provided; the temperature sensor may include a lead wire electrically connected to the control unit; the inner cylinder shaft may relatively be movable with respect to the outer cylinder shaft; and both the wiring and the lead wire may be attached to one and the same of the outer cylinder shaft and the inner cylinder shaft to extend in the liquid delivery path.

The present invention enables a surface temperature of a balloon to be highly precisely determined.

### Brief Description of The Drawings

Fig. 1 is a view for explaining one embodiment, showing a balloon catheter system and a balloon catheter.
Fig. 2 is a view showing a distal end portion of the balloon catheter of Fig. 1, with a balloon being inflated.
Fig. 3 is a view showing the distal end portion of the balloon catheter of Fig. 1, with the balloon being deflated and stretched.
Fig. 4 is a sectional view along a IV-IV line of Fig. 2.
Fig. 5 is a sectional view along a V-V line of Fig. 2.
Fig. 6 is temperature distribution at the distal end portion of the balloon catheter in a coaxial state, which was obtained by thermo-fluid analysis using CAE.
Fig. 7 is temperature distribution at the distal end portion of the balloon catheter in a non-coaxial state, which was obtained by thermo-fluid analysis using CAE.
Fig. 8 is a view showing the distal end portion of the balloon catheter, for explaining flow of a liquid when the liquid is injected from a liquid delivery path into the balloon.
Fig. 9 is a view showing the distal end portion of the balloon catheter, for explaining flow of a liquid when the liquid the balloon is sucked from the balloon to the liquid delivery path.
Fig. 10 is a view for explaining an experiment method using the balloon catheter system.
Fig. 11 is a view showing the distal end portion of the balloon catheter of Fig. 10.
Fig. 12 is a view showing the distal end portion of the balloon catheter in a coaxial state in the experiment of Fig. 10.
Fig. 13 is a view showing the distal end portion of the balloon catheter in a non-coaxial state in the experiment of Fig. 10.
Fig. 14 is a graph showing actual measured values of a temperature sensor and a surface temperature sensor, which were obtained in the experiment of Fig. 10 (liquid amount: 10 ml, contrast agent dilution ratio: 1:2, coaxial state).
Fig. 15 is a graph showing actual measured values of the temperature sensor and the surface temperature sensor, which were obtained in the experiment of Fig. 10 (liquid amount: 10 ml, contrast agent dilution ratio: 1:2, non-coaxial state).
Fig. 16 is a graph showing actual measured values of the temperature sensor and the surface temperature sensor, which were obtained in the experiment of Fig. 10 (liquid amount: 10 ml, contrast agent dilution ratio: 1:3, coaxial state).
Fig. 17 is a graph showing actual measured values of the temperature sensor and the surface temperature sensor, which were obtained in the experiment of Fig. 10 (liquid amount: 10 ml, contrast agent dilution ratio: 1:3, non-coaxial state).
Fig. 18 is a graph showing actual measured values of the temperature sensor and the surface temperature sensor, which were obtained in the experiment of Fig. 10 (liquid amount: 20 ml, contrast agent dilution ratio: 1:3, coaxial state).
Fig. 19 is a graph showing actual measured values of the temperature sensor and the surface temperature sensor, which were obtained in the experiment of Fig. 10 (liquid amount: 20 ml, contrast agent dilution ratio: 1:3, non-coaxial state).
Fig. 20 is a graph showing actual measured values of the temperature sensor and the surface temperature sensor, which were obtained in the experiment of Fig. 10 (liquid amount: 20 ml, contrast agent dilution ratio: 1:2, coaxial state).
Fig. 21 is a graph showing actual measured values of the temperature sensor and the surface temperature sensor, which were obtained in the experiment of Fig. 10 (liquid amount: 20 ml, contrast agent dilution ratio: 1:2, non-coaxial state).
Fig. 22 is a view showing a balloon catheter system used in a first specific example.
Fig. 23 is a circuit block diagram of a control unit used in the balloon catheter system of Fig. 22.
Fig. 24 is a flowchart showing a method of determining a surface temperature of a balloon from a detection result in the temperature sensor by the control unit of Fig. 23.
Fig. 25 is a graph showing actual measured values of the temperature sensor and the surface temperature sensor in an experiment where a balloon surface temperature is set at 66°C, which were determined from a detection result of the temperature sensor using the balloon system of Fig. 22.
Fig. 26 is a view showing a balloon catheter system used in a second specific example.
Fig. 27 is a flowchart showing a method of determining a surface temperature of a balloon from a detection result of the temperature sensor in a third specific example.

### Detailed Disclosure

An embodiment of the present invention will be described hereunder with reference to specific examples shown in the drawings. In the drawings attached to the specification, a scale dimension, an aspect ratio and so on are changed and exaggerated from the actual ones, for the convenience of easiness in illustration and understanding. In addition, terms used herein to specify shapes, geometric conditions and their degrees, e.g., "parallel", "orthogonal", "same", etc., and values of a length and an angle are not limited to their strict definitions, but construed to include a range capable of exerting a similar function, unless otherwise specified.

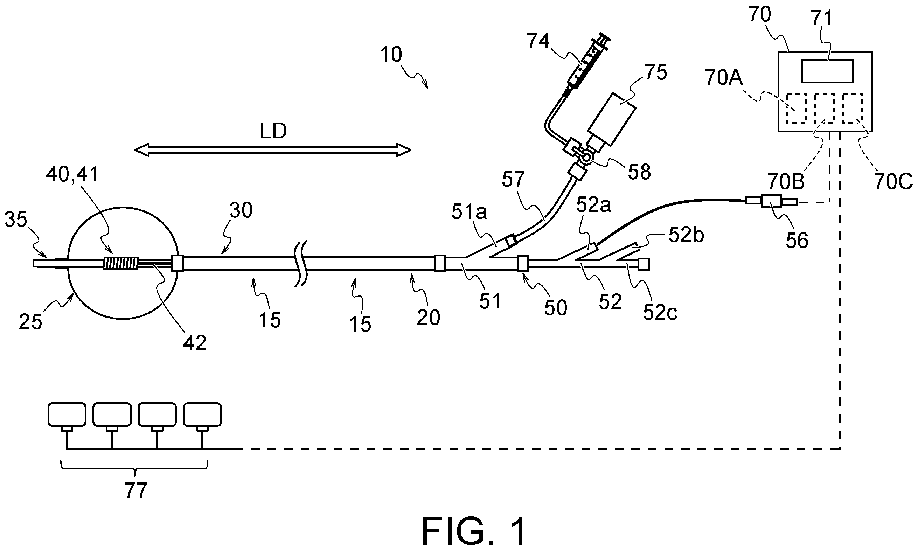

A balloon catheter system 10 shown in Fig. 1 has a balloon catheter 15, a control unit 70 connected to the balloon catheter 15, and an agitator 75 connected to the balloon catheter 15. The balloon catheter 15 also has a catheter body 20 having a longitudinal direction LD, and a handle 50 connected to a proximal end of the catheter body 20.

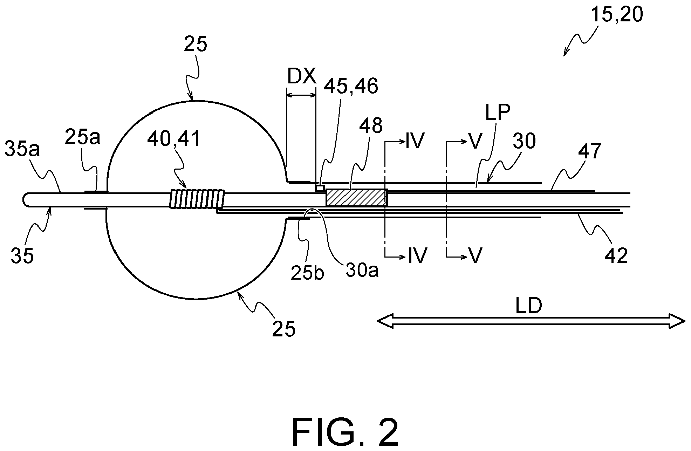

As shown in Fig. 2, the catheter body 20 according to this embodiment has a balloon 25, an outer cylinder shaft 30 connected to a proximal end 25b of the balloon 25, an inner cylinder shaft 35 connected to a distal end 25a of the balloon 25, and a heating component 40 disposed in the balloon 25. The inner cylinder shaft 35 passes through the outer cylinder shaft 30 to extend into the balloon 25. A liquid delivery path LP in communication with the balloon 25 is formed between the outer cylinder shaft 30 and the inner cylinder shaft 35. The heating component 40 heats a liquid in the balloon 25.

In particular, the catheter body 20 (balloon catheter 15) according to this embodiment is devised such that a surface temperature of the balloon 25 filled with a heated liquid can be highly precisely determined. Specifically, the temperature sensor 45 disposed in the liquid delivery path LP acquires information on a temperature in the liquid delivery path LP. Based on the information, a surface temperature of the balloon 25 can be highly precisely detected.

The longitudinal direction LD of the catheter body 20 is specified as a direction along which center axes of the outer cylinder shaft 30 and the inner cylinder shaft 35 extending from the outer cylinder shaft 30 extend. In this specification, the term "distal" side used for respective components of the balloon catheter 15 and the catheter body 20 means a side distant from an operator (physician) of the handle 50 and the balloon catheter 15 along the longitudinal direction LD of the catheter body 20, in other words, a distant side. In addition, the term "proximal" side used for respective components of the balloon catheter 15 and the catheter body 20 means a side close to the operator (physician) of the handle 50 and the balloon catheter 15 along the longitudinal direction LD of the catheter body 20, in other words, a near side.

The balloon catheter system 10 and the balloon catheter 15 are further described in detail hereunder. First, the catheter body 20 of the balloon catheter 15 is described in detail. As described above, the catheter body 20 of the balloon catheter 15 according to this embodiment has the balloon 25, the outer cylinder shaft 30, the inner cylinder shaft 35, the heating component 40, and the temperature sensor 45.

The outer cylinder shaft 30 and the inner cylinder shaft 35 both have a tubular shape, typically a circular tube or a circular cylindrical shape. Thus, the outer cylinder shaft 30 and the inner cylinder shaft 35 respectively form lumens as inside spaces. A not-shown guide wire, for example, is inserted thorough the lumen formed by the inner cylinder shaft 35. The inner cylinder shaft 35 is inserted through the lumen formed by the outer cylinder shaft 30. Namely, the outer cylinder shaft 30 and the inner cylinder shaft 35 form a dual shaft structure. An internal diameter of the outer cylinder shaft 30 is larger than an external diameter of the inner cylinder shaft 35. Thus, a lumen remains between the outer cylinder shaft 30 and the inner cylinder shaft 35. The lumen between the outer cylinder shaft 30 and the inner cylinder shaft 35 form the liquid delivery path LP. As shown in Fig. 2, the liquid delivery path LP is in communion with the balloon 25. The liquid delivery path LP extends up to an inside of the handle 50.

Lengths of the respective outer cylinder shaft 30 and the inner cylinder shaft 35 are preferably between 500 mm or more and 1700 mm or less, and more preferably between 600 mm or more and 1200 mm or less. The outer cylinder shaft 30 and the inner cylinder shaft 35 are preferably made of a flexible material with excellent antithrombotic properties. The flexible material with excellent antithrombotic properties may include, for example, fluoropolymers, polyamides, polyurethane-based polymers, or polyimides, but is not limited thereto. The outer cylinder shaft 30 is preferably manufactured by stacking layers of different flexible materials, in order to have both sliding facility to the inner cylinder shaft 35 and adhesion or heat weldability to the balloon 25.

The external diameter of the outer cylinder shaft 30 is preferably between 3.0 mm or more and 4.0 mm or less. The internal diameter of the outer cylinder shaft 30 is preferably between 2.5 mm or more and 3.5 mm or less. The external diameter of the inner cylinder shaft 35 is preferably between 1.4 mm or more and 1.7 mm or less. The internal diameter of the inner cylinder shaft 35 is preferably between 1.1 mm or more and 1.3 mm or less.

The balloon 25 is connected to the outer cylinder shaft 30 and the inner cylinder shaft 35. The balloon 25 is formed so as to be inflatable when it is filled with liquid and deflatable when a liquid is discharged therefrom. The balloon 25 is preferably shaped to fit a target site to be treated (e.g., blood vessel). As an example, the balloon 25 adapted for a pulmonary venous junction of a left atrium may have a spherical shape having a diameter between 15 mm or more and 40 mm or less. The spherical shape here includes a true spherical shape, a flatted spherical shape, and a prolate spheroid shape. It also includes a substantially spherical shape.

A film thickness of the balloon 25 is preferably between 10 µm or more and 200 µm or less. A material of the balloon 25 is preferably an elastic material with excellent antithrombotic properties. Specifically, a polyurethane-based polymer material can be used. The polyurethane-based polymer material applicable to the balloon 25 may be, for example, thermoplastic polyether urethane, polyether polyurethane urea, fluorinated polyether urethane urea, polyether polyurethane urea resin, or polyether polyurethane urea amide.

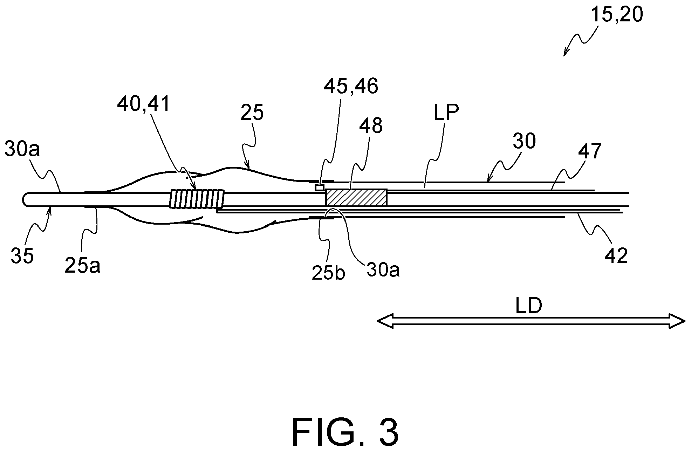

In the illustrated catheter body 20, as shown in Figs. 2 and 3, the distal end (distant end) 25a of the balloon 25 is fixed to a distal end (distant end) 35a of the inner cylinder shaft 35. The proximal end (near end) 25b of the balloon 25 is fixed to a distal end (distant end) 30a of the outer cylinder shaft 30. The balloon 25 may be connected to the outer cylinder shaft 30 and the inner cylinder shaft 35 by adhesion or heat welding.

By the relative movement of the outer cylinder shaft 30 and the inner cylinder shaft 35 in the longitudinal direction LD, the balloon 25 connected to the outer cylinder shaft 30 and the inner cylinder shaft 35 deforms. In the illustrated example, the relative movement of the outer cylinder shaft 30 and the inner cylinder shaft 35 allows a dimension of the balloon 25 to be adjusted in the longitudinal direction LD. As shown in Fig. 3, when the inner cylinder shaft 35 is relatively moved with respect to the outer cylinder shaft 30 to the distal side in the longitudinal direction LD, the balloon 25 is stretched in the longitudinal direction LD and is strained. In the illustrated example, the movement range of the inner cylinder shaft 35 with respect to the outer cylinder shaft 30 to the distal side in the longitudinal direction is restricted by the balloon 25. When the inner cylinder shaft 35 is relatively moved with respect to the outer cylinder shaft 30 from the position shown in Fig. 3 to the proximal side in the longitudinal direction LD, the balloon 25 becomes loosened. By introducing a liquid into the loosened balloon 25, as shown in Fig. 2, the balloon 25 can be inflated. Namely, the relative movement of the outer cylinder shaft 30 and the inner cylinder shaft 35 allows for the dimension of the balloon 25 to be adjusted in the longitudinal direction LD.

Next, the heating component 40 is described. The heating component 40 is disposed in the balloon 25. The heating component 40 is a member for heating a liquid filled in the balloon 25. As an example, a nichrome wire that generates electric resistance heat can be employed as the heating component 40. A coil electrode 41 may be employed as another example of the heating component 40, as shown in Figs. 2 and 3. By means of high-frequency current conduction (high-frequency energization) to the heating component 40 as the coil electrode 41, a high-frequency current flows between the coil electrode 41 and a return pad 77 (Fig. 1) disposed outside, so that the liquid positioned between the coil electrode 41 and the return pad 77 generates Joule heat. The return pad 77 is located on the back of a patient, for example.

In the example shown in Figs. 2 and 3, the coil electrode 41 is provided on the inner cylinder shaft 35 extending inside the balloon 25. The coil electrode 41 may be made by a conductive wire wound around the inner cylinder shaft 35. The coil electrode 41 is electrically connected to a wiring 42 for high-frequency current conduction. The wiring 42 extends in the liquid delivery path LP, which serves as a lumen between the outer cylinder shaft 30 and the inner cylinder shaft 35, up to the handle 50. A specific example of the coil electrode 41 forming the heating component 40 may be a coil electrode that is made as follows. An insulation-coated lead wire used as the wiring 42 is stripped of its coating. Then, the lead wire is wound around the inner cylinder shaft 35 to provide a coil electrode. Since such a coil electrode 41 is integrally formed with the wiring 42, occurrence of trouble such as disconnection or open circuit of wire can be effectively suppressed.

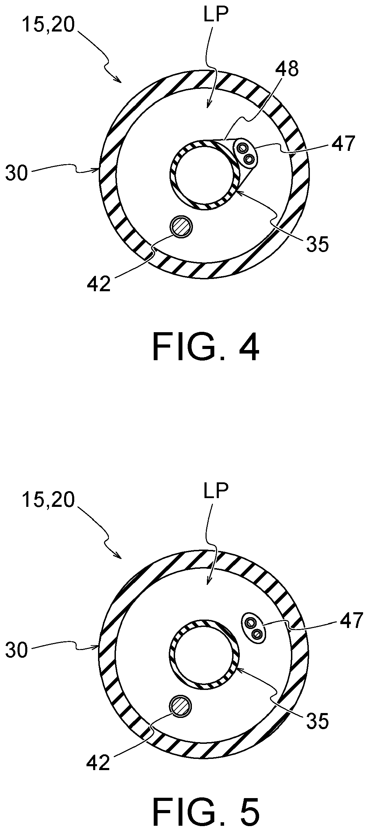

Diameters of the coil electrode 41 and the wiring 42 are preferably between 0.1 mm or more and 1 mm or less, and more preferably between 0.1 mm or more and 0.4 mm or less. A conductive material forming the coil electrode 41 and the wiring 42 may be, for example, copper, silver, gold, platinum, and alloys thereof. In order to prevent a short circuit, a conductive liner portion of the wiring 42 is preferably covered with an insulating film made of fluoropolymer, for example (see Figs. 4 and 5).

Next, the temperature sensor 45 is described. The temperature sensor 45 acquires information on a liquid temperature. In this embodiment, the temperature sensor 45 has a thermosensitive part 46 disposed in the liquid delivery path LP positioned between the outer cylinder shaft 30 and the inner cylinder shaft 35. The temperature sensor 45 can acquire information on a liquid temperature in the liquid delivery path LP. According to the inventors' study, a surface temperature of the balloon 25, which is important in ablation therapy using the balloon catheter system 10, can be highly precisely determined based on the information acquired by the temperature sensor 45. Installation of the temperature sensor 45 between the outer cylinder shaft 30 and the inner cylinder shaft 35 can greatly simplify the manufacture of the catheter body 20, as compared with installation of the temperature sensor inside the balloon 25. In addition, the temperature sensor 45 located between the outer cylinder shaft 30 and the inner cylinder shaft 35 can be more securely protected from an external stress and can be more stably supported, as compared with a case in which the temperature sensor 45 is located within the balloon 25 which has a very small thickness and is greatly deformed during use. Namely, installation of the temperature sensor 45 in the liquid delivery path LP between the outer cylinder shaft 30 and the inner cylinder shaft 35 can significantly improve the quality and reliability of the balloon catheter system 10 and the balloon catheter 15.

In order to highly precisely determine a surface temperature of the balloon 25, there is a preferable length DX along the longitudinal direction LD of the outer cylinder shaft from the distal end 30a of the outer cylinder shaft 30 up to the thermosensitive part 46 of the temperature sensor 45. In a strict sense, the preferable length DX depends on an amount of liquid that is supplied and discharged by the agitator 75 described below. However, in consideration of respective dimensions of the balloon catheter 15 generally applied to a cardiac ablation therapy and an amount of liquid supplied and discharged by the agitator 75, the length DX from the distal end 30a of the outer cylinder shaft 30 up to the temperature sensor 45 (see Fig. 2) is preferably between 5 mm or more and 150 mm or less, and more preferably between 10 mm or more and 20 mm or less.

The length DX from the distal end 30a of the outer cylinder shaft 30 up to the temperature sensor 45 means a length that is measured when the balloon 25 shown in Fig. 2 is inflated with a liquid, unless otherwise specified. Similarly, unless otherwise specified, the expressions like "the temperature sensor 45 is poisoned in the liquid delivery path LP", "the temperature sensor 45 is positioned in the outer cylinder shaft 30" and "the temperature sensor 45 is positioned between the outer cylinder shaft 30 and the inner cylinder shaft 35" assume that the balloon 25 shown in Fig. 2 is inflated with a liquid.

A thermocouple or thermistor can be used as the temperature sensor 45. In particular, a T-type thermocouple is suitable as the temperature sensor 45. The T-type thermocouple allows reduction in heat capacity of the thermosensitive part 46. In addition, employment of the T-type thermocouple as the temperature sensor 45 stabilizes a thermal electromotive force. Further, since the T-type thermocouple can highly precisely detect a temperature range between 50°C or more and 80°C or less, it is particularly suitable for a cardiac ablation therapy. Information on a temperature acquired by the temperature sensor 45 is, for example, an electric potential that can be acquired from a thermocouple, or a resistance value that can be acquired from a thermistor.

As shown in Figs. 2 and 3, the temperature sensor 45 typically has the thermosensitive part 46, and a lead wire 47 electrically connected to the thermosensitive part 46. When the temperature sensor 45 comprises a thermocouple, a part where different metals are connected serves as the thermosensitive part 46. When the temperature sensor 45 comprises a thermistor, a ceramic element serves as the thermosensitive part 46. The lead wire 47 extends inside the liquid delivery path LP, which serves as a lumen between the outer cylinder shaft 30 and the inner cylinder shaft 35, up to the handle 50.

A diameter of the lead wire 47 is preferably between 0.05 mm or more and 0.5 mm or less, and more preferably between 0.05 mm or more and 0.3 mm or less. When the temperature sensor 45 comprises a thermocouple, copper may be used in one lead wire 47 and constantan may be used in the other lead wire 47, for example. In this example, the thermosensitive part 46 where the pair of lead wires 47 are joined can function as a T-type thermocouple. In order to prevent a short circuit of the pair of lead wires 47, they are preferably provided with an electrically insulating coat made of fluoropolymer, enamel, etc., as shown in Figs. 4 and 5.

In the illustrated example, the temperature sensor 45 is attached to the inner cylinder shaft 35. As shown in Figs. 2 to 4, the temperature sensor 45 is attached to the inner cylinder shaft 35 by fixing the lead wire 47 of the temperature sensor 45. The thermosensitive part 46 is apart from both the outer cylinder shaft 30 and the inner cylinder shaft 35. In other words, the thermosensitive part 46 is not in contact with the outer cylinder shaft 30 and the inner cylinder shaft 35. Thus, responsiveness of the temperature sensor 45 can be avoided from being deteriorated by temperatures of the outer cylinder shaft 30 and the inner cylinder shaft 35 having large heat capacities. This makes it possible to highly precisely evaluate a liquid temperature in the liquid delivery path LP using the temperature sensor 45 with excellent responsiveness. Various means may be used without particular limitation as fixing means 48 for fixing the lead wire 47 to the inner cylinder shaft 35. In the illustrated example, a heat shrinkable tube that shrinks upon being heated is used as the fixing means 48. However, not limited to this example, various members such as a shrinkable tube, an adhesive tape, an adhesive agent, etc. may be used as the fixing means 48.

In the illustrated example, the wiring 42 is attached neither to the outer cylinder shaft 30 nor to the inner cylinder shaft 35. However, not limited to this example, the wiring 42 may be attached either the outer cylinder shaft 30 or the inner cylinder shaft 35. Preferably, both the wiring 42 and the lead wire 47 of the temperature sensor 35 are attached to one and the same of the outer cylinder shaft 30 and the inner cylinder shaft 35. This specific example can effectively prevent entanglement of the wiring 42 and the lead wire 47 both of which extend inside the liquid delivery path LP, when the outer cylinder shaft 30 and the inner cylinder shaft 35 are moved relatively to each other. This allows stable adjustment of a liquid temperature in the balloon by means of the heating component 40, and stable understanding of a surface temperature of the balloon 25.

As shown in Fig. 3, even when the inner cylinder shaft 35 is relatively moved with respect to the outer cylinder shaft 30 to the distal side in the longitudinal direction LD to a maximum extent, the temperature sensor 45 is positioned in the outer cylinder shaft 30. This specific example allows the temperature sensor 45 to be position in the outer cylinder shaft 30, regardless of a relative position of the inner cylinder shaft 35 with respect to the outer cylinder shaft 30. Thus, the temperature sensor 45 can be stably protected by the outer cylinder shaft 30, regardless of a relative position of the inner cylinder shaft 35 with respect to the outer cylinder shaft 30.

On the other hand, unlike the illustrated example, the temperature sensor 45 may be attached to the outer cylinder shaft 30. For example, the lead wire 47 of the temperature sensor 45 may be fixed to an inner surface of the outer cylinder shaft 30. This specific example allows the temperature sensor 45 to be poisoned in the outer cylinder shaft 30, regardless of a relative position of the inner cylinder shaft 35 with respect to the outer cylinder shaft 30. Thus, the temperature sensor 45 can be stably protected by the outer cylinder shaft 30, regardless of a relative position of the inner cylinder shaft 35 with respect to the outer cylinder shaft 30.

Next, the handle 50 connected to the catheter body 20 from the proximal side is described. The handle 50 is a part grasped by an operator (physician) during the use of the balloon catheter system 10. Thus, the handle 50 preferably has a design that allows an operator to easily grasp and operate the handle 50 by his/her hand. A material forming the handle 50 preferably has excellent chemical resistance. For example, polycarbonate or ABS resin can be used.

The handle 50 shown in Fig. 1 has a first handle part 51 and a second handle part 52 that are slidable to each other. The first handle part (front handle part) 51 is connected to the outer cylinder shaft 30 of the catheter body 20. The second handle part (rear handle part) 52 is connected to the inner cylinder shaft 35 of the catheter body 20. By relatively moving the second handle part 52 with respect to the first handle part 51, the inner cylinder shaft 35 can be relatively moved with respect to the outer cylinder shaft 30.

As shown in Fig. 1, the handle 50 also functions as a part that connects other devices included in the balloon catheter system 10 and the balloon catheter 15.

A connector 56 extends from the second handle part 52. This connector 56 electrically connects the wiring 42 of the catheter body 20 and the lead wire 47 of the temperature sensor 45 to the external control unit 70. The connector 56 extends from one (branch 52a) of the branches of the second handle part 52. When the wiring 42 and the lead wire 47 are connected to an external device (control unit 70) through the same handle part, the wiring 42 and the lead wire 47 are preferably attached to one and the same of the outer cylinder shaft 30 and the inner cylinder shaft 35, as described above, in particular, to the shaft (the inner cylinder shaft 35 in the illustrated example) connected to this handle part (the second handle part 52 in the illustrated example). In this case, entanglement and/or disconnection of the wire 42 and the lead weir 47 can be move effectively avoided.

The connector 56 preferably has a structure capable of effectively preventing improper connection. In addition, the connector 56 preferably has excellent waterproofness. The structure of the connector 56 can be decided in consideration of physician's convenience and design matter. Similarly to the handle 50, a material forming the connector 56 preferably has excellent chemical resistance. For example, polycarbonate or ABS resin can be used.

The connector 56 may have therein a highly conductive metal pin. The wire 42 and the lead wire 47 are connected to this highly conductive metal pin so as to be electrically connected to the control unit 70 as means for supplying high-frequency power. Note that the lead wire 47 of the temperature sensor 45 may be electrically connected to a device other than the control unit 70 as means for supplying high-frequency power, such as a temperature indicator. A material of the highly conductive metal pin included in the connector 56 may be of any type, as long as it is a metal having high conductivity. A material of the high conductive metal pin included in the connector 56 may be, for example, copper, silver, gold, platinum, and alloys thereof. In addition, an outside of the highly conductive metal pin is preferably protected by a material having electrically insulating properties and chemical resistance. An electrically insulating and chemically resistant material may be, for example, polysulfone, polyurethane polymers, polypropylene, or polyvinyl chloride.

The second handle part 52 has branches 52b and 52c other than the branch 52a to which the connector 56 is connected. These branches 52b and 52c function as a part through which a liquid is supplied to the lumen as an inside space of the inner cylinder shaft 35, and a part from which a guide wire inserted through the lumen of the inner cylinder shaft 35 extends. During a cardiac ablation therapy, a physiological saline solution an amount of which is as small as about 100 ml/hour is generally injected into a body through the lumen of the inner cylinder shaft 35. The injection of physiological saline solution effectively prevents backflow of blood into the lumen of the inner cylinder shaft 35.

In addition, as shown in Fig. 1, an extension tube 57 extends from the first handle part 51. The extension tube 57 communicates the liquid delivery path LP of the catheter body 20 with an external supply unit 74 and the agitator 75. The extension tube 57 extends from the branch 51a provided on the first handle part 51. The extension tube 57 is connected to the supply unit 74 and the agitator 75 through a valve 58. In the illustrated example, whether the supply unit 74 or the agitator 75 is communicated with the liquid delivery path LP can be selected by operating the valve 58. A three-way stopper cock may be used as the valve 58.

Next, devices constituting the balloon catheter system 10 together with the aforementioned balloon catheter 15, to be specific, the control unit 70, the supply unit 74 and the agitator 75, are described.

The illustrated control unit 70 is electrically connected to the coil electrode 41 through the wiring 42. The control unit 70 has a high-frequency current conduction controller 70A that controls high-frequency current conduction (high-frequency voltage supply) to the coil electrode 41. In the illustrated example, the high-frequency current conduction controller 70A controls the high-frequency current conduction to/through the coil electrode 41 to adjust an output from the heating component 40. The high-frequency current conduction controller 70A can control the high-frequency current conduction to the coil electrode 41 based on a surface temperature of the balloon 25 determined by a temperature calculation unit 70B described later, or in accordance with a preset process, or in accordance with an input from an operator.

In addition, the control unit 70 is electrically connected to the lead wire 47 of the temperature sensor 45. The control unit 70 has the temperature calculation unit 70B that process information on a temperature acquired by the inner cylinder shaft 35. The temperature calculation unit 70B calculates a liquid temperature in the liquid delivery path LP based on information on a temperature acquired by the temperature sensor 45, and further estimates a surface temperature of the balloon 25 based on the calculated liquid temperature. The temperature calculation unit 70B may display a determined surface temperature of the balloon 25 on a display 71. How to determine a surface temperature of the balloon 25 is described in detail later.

The control unit 70 further has an agitator controller 70C that controls the agitator 75. The agitator controller 70C may display a control condition of the agitator 75 on the display 71.

The control unit 70 is constituted by a hardware such as a CPU, for example, One or more of the high-frequency current conduction controller 70A, the temperature calculation unit 70B and the agitator 70C, which are included in the control unit 70, may be constituted by a separate or same hardware. At least a part of the control unit 70 may be constituted by a software. A part of the control unit 70 may be physically separated therefrom. A component of the control unit 70 may be able to cooperate with another component thereof by communication through a network. A component of the control unit 70 may be on a device, such as a server or database in the cloud, which can communicate with another component through an external network.

Next, the supply unit 74 is described. The supply unit 74 supplies a liquid into the liquid delivery path LP. By supplying a liquid from the supply unit 74 to the balloon 25 through the liquid delivery path LP, the balloon 25 can be inflated as shown in Fig. 2. On the other hand, by discharging the liquid from the supply unit 74 from the balloon 25 through the liquid delivery path LP, the balloon 25 can be deflated. The liquid to be supplied into the liquid delivery path LP may typically be a physiological saline solution. A syringe can be used as the supply unit 74 as illustrated. However, a pump or the like can also be used as the supply unit 74.

Next, the agitator 75 is described. The agitator 75 is provided for agitating a liquid in the balloon 25. By agitating the liquid in the balloon 25, heat supplied into the balloon 25 can be distributed or equalized so that a surface temperature of the balloon 25 can be adjusted. The agitator 75 repeats supply of liquid to the liquid delivery path LP and discharge of liquid from the liquid delivery path LP. A pump selected from the group consisting of a roller pump, a diaphragm pump, a bellows pump, a vane pump, a centrifugal pump, and a pump comprising a piston and a cylinder in combination, can be employed as the agitator 75.

An amount of liquid to be supplied to the liquid delivery path LP and an amount of liquid to be discharged from the liquid deliver path LP may be a predetermined amount (e.g., between 5 ml or more and 30 ml or less). Supply of liquid to the liquid delivery path LP and discharge of liquid from the liquid delivery path LP may be repeated at a regular cycle (e.g., between once or more and five times or less per second). An amount of liquid to be supplied to the liquid delivery path LP and an amount of liquid to be discharged from the liquid deliver path LP may be adjusted by a control signal from the aforementioned agitator controller 70C or by a direct input from an operator. Similarly, a cycle at which a liquid is supplied to the liquid delivery path LP and a cycle at which a liquid is discharged from the liquid delivery path LP may be adjusted by a control signal from the aforementioned agitator controller 70C or by a direct input from an operator.

Next, an example of the use of the balloon catheter system 10 as structured above is described.

The valve 58 is operated first such that the supply unit 74 communicates with the liquid delivery path LP of the catheter body 20 through the handle 50. Then, the supply unit 74 is operated to let a liquid to flow into the liquid delivery path LP, so that the balloon 25, the liquid delivery path LP and the extension tube 57 are filled with the liquid. Thereafter, the inner cylinder shaft 35 is relatively moved with respect to the outer cylinder shaft 30 to the distal side (distant side) in the longitudinal direction LD, so that the balloon 25 is stretched as shown in Fig. 3. At this time, by operating the first handle part 51 and the second handle part 52 of the handle 50, the outer cylinder shaft 30 and the inner cylinder shaft 35 can be relatively moved to each other. Then, the catheter body 20 with the balloon 25 stretched therealong is inserted into a body.

When the distal end of the catheter body 20 has been guided close to a target site (affected area), the inner cylinder shaft 35 is relatively moved with respect to the outer cylinder shaft 30 to the proximal side (near side) in the longitudinal direction LD, so that the balloon 25 is loosened. Thereafter, the valve 58 is operated to communicate the supply unit 74 with the liquid delivery path LP of the catheter body 20 through the handle 50. Then, the supply unit 74 is operated to let a liquid to flow into the liquid delivery path LP, so that the balloon 25 is inflated with the liquid as shown in Fig. 2.

Then, the valve 58 is operated to shut off the supply unit 74 from the liquid delivery path LP, and to communicate the agitator 75 with the liquid delivery path LP. The agitator 75 is controlled by a control signal from the agitator controller 70C of the control unit 70. The agitator 75 repeats supply of a predetermined amount of liquid to the liquid delivery path LP and discharge of a predetermined amount of liquid from the liquid delivery path LP at a regular cycle. This results in repeated injection of the predetermined amount of liquid from the liquid delivery path LP into the balloon 25 and suction of the predetermined amount of liquid from inside the balloon 25 to the liquid delivery path LP at the regular cycle. Thus, the liquid in the balloon 25 is agitated.

A liquid temperature in the balloon 25 is adjusted by controlling the heating component 40 by means of the high-frequency current conduction controller 70A of the control unit 70. Specifically, high-frequency voltage is applied from the control unit 70 to between the coil electrode 41 forming the heating component 40 and the return pad 77 disposed outside the body of a patient. As a result, a high-frequency current is generated between the coil electrode 41 and the current electrode 77. Since the size of the coil electrode 41 is significantly larger than that of the current electrode, a current density around the coil electrode 41 increases, and the liquid and a contrast agent around the coil electrode 41 are heated by Joule heating.

The temperature sensor 45 is disposed close to the coil electrode 41. Note that the temperature sensor 45 is disposed not in the balloon 25 but in the outer cylinder shaft 30 having a thickness much larger than that of the balloon 25. Thus, the temperature sensor 45 can be shielded from the high-frequency current by the outer cylinder shaft 30, whereby local increase in temperature of the temperature sensor 45 and the liquid around the temperature sensor 45 can be effectively avoided under the influence of the high-frequency current. Namely, detection of abnormal values by the temperature sensor 45 can be effectively prevented.

The liquid in the balloon 25 is heated and agitated as described above. Then, the balloon 25 containing the heated liquid is brought into contact with the target site to ablate the target site. During the ablation, the temperature sensor 45 disposed in the liquid delivery path LP acquires information on a liquid temperature in the liquid delivery path LP. The acquired information is processed by the temperature calculation unit 70B of the control unit 70. In particular, the temperature calculation unit 70B not only can merely determine a temperature of the liquid in a region where the thermosensitive part 46 of the temperature sensor 45 is disposed, but also can highly precisely determine a surface temperature of the balloon 25 as described later. The surface temperature highly precisely determined by the temperature calculation unit 70B can be displayed on the display 71, for example.

Namely, the use of the balloon catheter system 10 allows an operator to perform an ablation therapy while exactly understanding a surface temperature of the balloon 25 at all times. Thus, the operator can proceed the operation while adjusting a surface temperature of the balloon 25, which is most important in the ablation therapy, to an ideal temperature. This can significantly improve the ablation therapy effect.

Upon completion of the ablation to the target site, energy supply to the heating component 40 is stopped. In addition, the valve 58 is operated so that the supply unit 74 is communicated with the liquid delivery path LP of the catheter body 20 through the handle 50, and that the agitator 75 is shut off from the liquid delivery path LP. Then, the liquid is discharged by means of the supply unit 74 from the liquid delivery path LP to deflate the balloon 25. Then, the second handle part 52 is operated to stretch the deflated balloon 25 as shown in Fig. 3. After that, the catheter body 20 with the balloon 25 stretched therealong is pulled out from the body. In this manner, the procedure using the balloon catheter system 10 is completed.

Next, how the temperature sensor 45, which has the thermosensitive part 46 disposed between the outer cylinder shaft 30 and the inner cylinder shaft 35 and acquires information on a temperature in the liquid delivery path LP, can highly precisely detect a surface temperature of the balloon 25 is further described in detail.

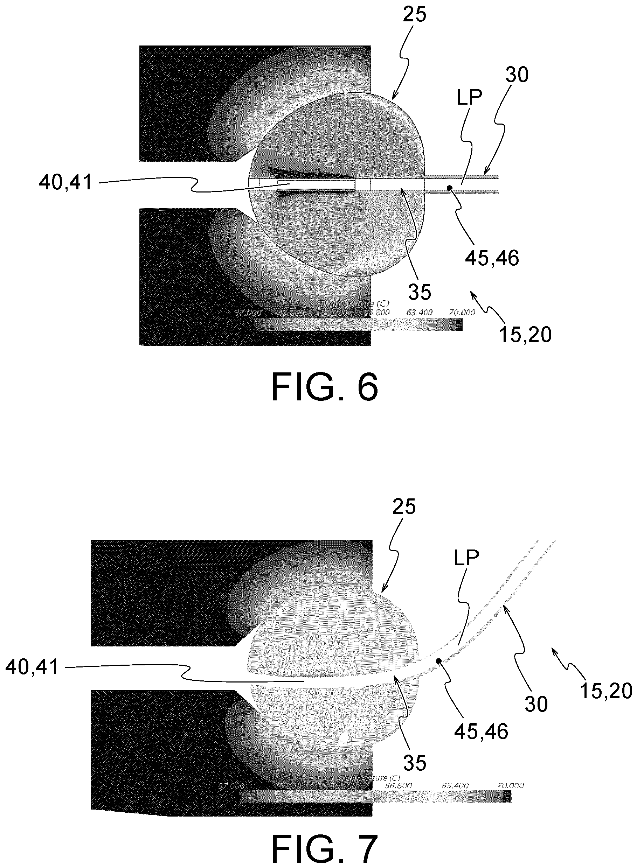

Figs. 6 and 7 show results of temperature distributions in the balloon 25 by CAE (computer aided engineering). Fig. 6 is a simulation result where the balloon 25 is pressed against the target site along the longitudinal direction LD (also referred to merely as "coaxial state" hereunder). In the example shown in Fig. 6, the outer cylinder shaft 30 substantially aligns with the inner cylinder shaft 35 and the heating component 40 in the balloon 25. On the other hand, Fig. 7 is a simulation result where the balloon 25 is pressed against the target site from a direction inclined to the longitudinal direction LD (also referred to merely as "non-coaxial state" hereunder). In the example shown in Fig. 7, the outer cylinder shaft 30 is greatly inclined with respect to the inner cylinder shaft 35 and the heating component 40 in the balloon 25.

These simulation results show that, even when the liquid in the balloon 25 is agitated by means of the agitator 75, the liquid in the balloon 25 has a temperature gradient because of the position of the heating component 40. When the coil electrode 41 is used as the heating component 40, the temperature distribution has a tendency similar to distribution of current density. In the coaxial state shown in Fig. 6, the temperature distribution of 5°C or more occurs in the balloon 25. In the non-coaxial state shown in Fig. 7, the temperature distribution of about 5°C occurs in the balloon 25. Namely, since the temperature distribution in the balloon 25 differs depending on the pressing state of the balloon 25 against the target site, it is difficult to determine a surface temperature of the balloon 25 based only on information from a temperature sensor disposed close to the heating component 40 in the balloon 25.

On the other hand, a temperature of the liquid in a region close to the distal end 30a of the outer cylinder shaft 30 where the temperature sensor 45 is located is different from the liquid temperature around the heating component 40 but is substantially the same as a surface temperature of the balloon 25 both in the coaxial state shown in Fig. 6 and in the non-coaxial state shown in Fig. 7. It can be understood first that a surface temperature of the balloon 25 can be highly precisely detected by disposing the thermosensitive part 46 of the temperature sensor 45 between the outer cylinder shaft 30 and the inner cylinder shaft 35.

Not only the heating component 40 expected to generate resistance heating by a nichrome wire, but also the heating component 40 having the coil electrode 41 for high-frequency current conduction have the temperature gradients around the heating component 40 in the balloon 25, as verified by the simulation results. When the high-frequency voltage is applied to the coil electrode 41 and the return pad (opposite electrode) 77 outside the body, a high-frequency current flows between the coil electrode 41 and the return pad 77. A specific resistance of the liquid close to the coil electrode 41 is high. Thus, the high-frequency current generates Joule heat intensively around the coil electrode 41. The Joule heat is represented as (Value of flowing current) ² × (Resistance value of filling liquid). The Joule heat decreases rapidly as it moves away from the coil electrode 41. This is because the term "(Value of flowing current)" in "(Value of flowing current) ² × (Resistance value of filling fluid)" decreases with the square of the distance. For example, when the liquid temperature close to the heating component 40 in the simulation result of Fig. 6 is 70 degrees, the balloon surface temperature decreases as low as 65°C or less.

Next, effect of agitation by the agitator 75 that repeats liquid supply and liquid discharge on a temperature of the liquid was studied and experimented. The study and experiment conducted by the inventors are described hereunder.

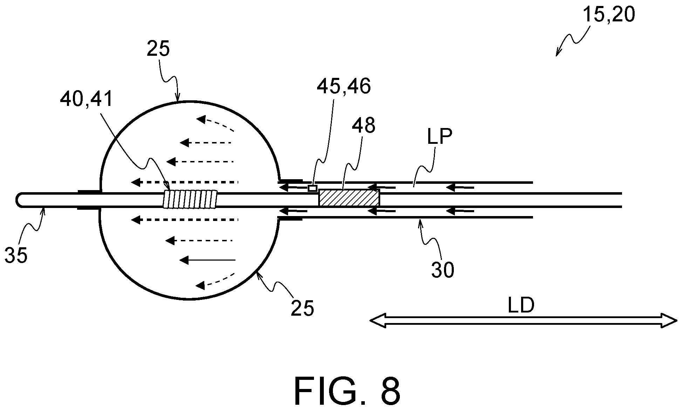

When the liquid is supplied from the agitator 75 to the liquid delivery path LP, the liquid is injected into the balloon 25 from the distal end 30a of the outer cylinder shaft 30, as shown in Fig. 8. Thus, the liquid in the balloon 25 is agitated, and the liquid around the heating component 40, which has relatively a high temperature, moves to the surface of the balloon 25. Particularly in the illustrated example, the distal end 30a of the outer cylinder shaft 30 is opened toward the heating component 40. Thus, the liquid is ejected from the distal end 30a of the outer cylinder shaft 30 toward the heating component 40. This allows the heat around the heating component 40 to be efficiently diffused in the balloon 25.

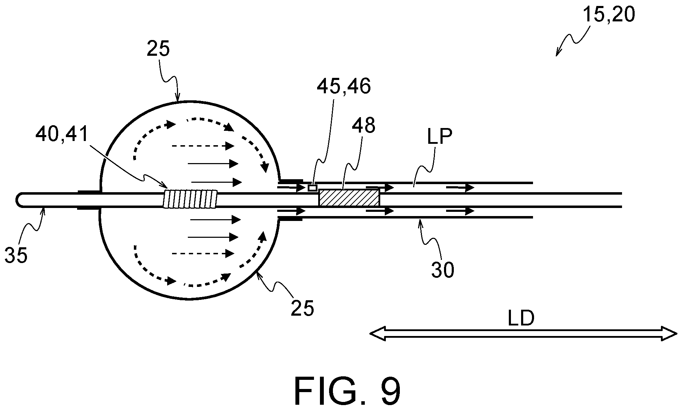

On the other hand, when the agitator 75 discharges the liquid from the liquid delivery path LP, the liquid in the balloon 25 is sucked into the outer cylinder shaft 30, as shown in Fig. 9. At this time, the liquid having a high temperature in the balloon 25 flows into the liquid delivery path LP. The intensive study of the inventors shows as follows. When the liquid is sucked from inside the balloon 25 having substantially a spherical shape to the liquid delivery path LP immediately after or in parallel with discharge of liquid from the outer cylinder shaft 30 toward the heating component 40 in the center of the substantially spherical balloon 25, the liquid having flown along the surface of the balloon 25 tends to flow into the liquid delivery path LP. Thus, according to this embodiment, a surface temperature of the balloon 25 is considered to be highly precisely determined by measuring a temperature of the liquid having flown into the liquid delivery path LP.

From this point of view, the thermosensitive part 46 of the temperature sensor 45 is preferably located close to the distal end 30a of the outer cylinder shaft 30. More specifically, the thermosensitive part 46 is preferably located in a region of the liquid delivery path LP close to the distal end 30a of the outer cylinder shaft 30, to which the liquid in the balloon 25 is drawn when the liquid is sucked by the agitator 75. This enables a temperature of the liquid, which was positioned close to the surface of the balloon 25 until just before the suction, to be detected by the temperature sensor 45 in the outer cylinder shaft 30. Specifically, a value of a length DX from the distal end 30a of the outer cylinder shaft 30 up to the temperature sensor 45 (see Fig. 2) is preferably equal to or less than a value obtained by dividing an amount [mm³] of liquid discharged by the agitator 75 from the liquid delivery path LP, by a cross-sectional area [mm²] of the liquid delivery path LP.

Further, from the study shown in Figs. 8 and 9, a temperature of the liquid detected by the temperature sensor 45 differs between a state where a liquid is injected into the balloon 25, which is shown in Fig. 8, and a state where a liquid is drawn from inside the balloon 25, which is shown in Fig. 9. Specifically, in the state shown in Fig. 8, the temperature sensor 45 detects a temperature of the liquid which was located on the proximal side of the temperature sensor 45 and thus was not yet heated by the heating component 40, the temperature sensor 45 detects a lower temperature. On the other hand, in the state shown in Fig. 9, since the temperature sensor 45 measures the liquid which was located in the balloon 25, the temperature sensor 45 detects a higher temperature. In particular, in consideration of the liquid flow in the balloon 25, a temperature, which is measured by the temperature sensor 45 in the state shown in Fig. 9, reflects a surface temperature of the balloon 25 more precisely. These points are verified by the inventors' experiment results described later.

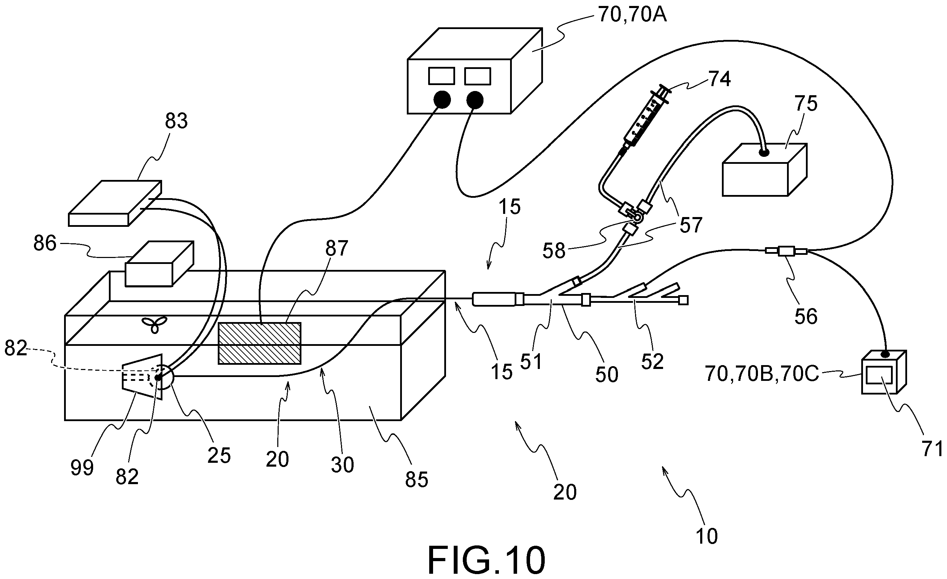

Fig. 10 shows a balloon catheter system 10 used in an experiment conducted for confirming an effect of agitation by the agitator 75. This experiment compared an actual measured value of a surface temperature of the balloon 25, and an actual measured value of a temperature of a liquid in the liquid delivery path LP, which was determined by information acquired by the temperature sensor 45. This experiment used the aforementioned balloon catheter system 10 shown in Fig. 1. Note that the catheter body 20 was provided with an electrode temperature sensor 81 for measuring a temperature in an immediate vicinity of the coil electrode 41, and a surface temperature sensor 82 for directly measuring a temperature of a surface of the balloon 25.

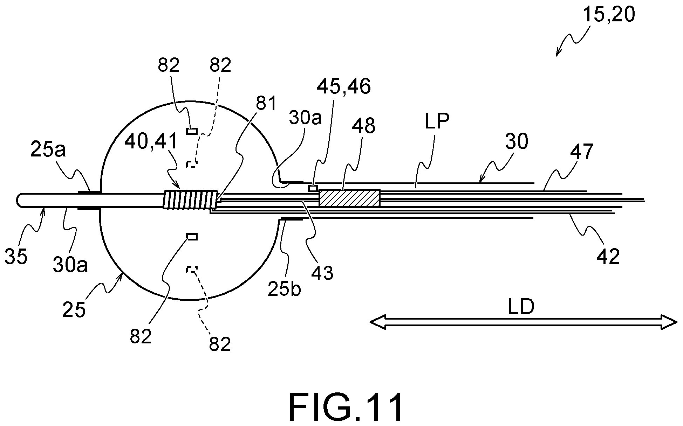

As shown in Fig. 11, the electrode temperature sensor 81 was composed of the coil electrode 41, and an electrode temperature sensor wiring 43 sandwiched between the coil electrode 41 and the inner cylinder shaft 35. A detection result of the electrode temperature sensor 81 was imported to the temperature calculation unit 70B of the control unit 70. The high-frequency current conduction controller 70A received a calculation result of the temperature calculation unit 70B, and controlled high-frequency current conduction to the coil electrode 41 based on information acquired by the electrode temperature sensor 81. Fig. 10 omits illustration of the electrode temperature sensor 81.

A T-type thermocouple having a film shape (shape: about 5 × 15 mm, thickness: about 0.1 mm) was used as the surface temperature sensor 82. Four surface temperature sensors 82 were applied to the balloon surface by using a polyimide tape of 0.1 mm in thickness. As shown in Fig. 11, the four surface temperature sensors 82 were located on center positions of the balloon 25 in the longitudinal direction LD. The four surface temperature sensors 82 were located on the balloon 25 so as to be evenly spaced in the circumferential direction about the central axis of the inner cylinder shaft 35. The four surface temperature sensors 82 were electrically connected a high-precision temperature logger 83 (manufacturer: HIOKI, model number: LR8431), and a surface temperature was determined by the temperature logger 83 based on information acquired by the surface temperature sensors 82. An average value of the values determined by the four surface temperature sensors 82 was defined as an actual measured value of a surface temperature of the balloon 25. In Fig. 10, only two surface temperature sensors 82 are illustrated and the remaining two are omitted.

In this experiment, an ablation therapy was applied to a pseudo-living body 99 that mimicked a Left atrium pulmonary vein ostium of a body. The pseudo-living body 99 was immersed in a physiological saline solution held in a water tank 85. During the experiment, the physiological saline solution in the water tank 85 was agitated using a water-tank agitator 86. A return pad 87, which generates a high-frequency current between it and the coil electrode 41 of the catheter body 20, was located on a sidewall of the water tank 85. The physiological saline solution in the water tank 85 was formed by dissolving 0.9 wt% salt (sodium chloride) in water.

A liquid supplied from the supply unit 74 to the liquid delivery path LP and the balloon 25 was formed by mixing a physiological saline solution in which 0.9 wt% salt (sodium chloride) was dissolved in water, with a contrast agent for X-ray contrast. An injection amount of liquid into the balloon 25 had two levels, i.e., 10 mL and 20 mL which are generally used in actual ablation therapy. The contrast agent mixed in the liquid was Omnipark (registered trademark) manufactured by Daiichi Sankyo Company, Ltd. A dilution ratio of the contrast agent (contrast agent dilution ratio) had two levels, i.e., 1:2 and 1:3. The contrast agent dilution ratio here means "physiological saline solution volume: contrast agent volume".

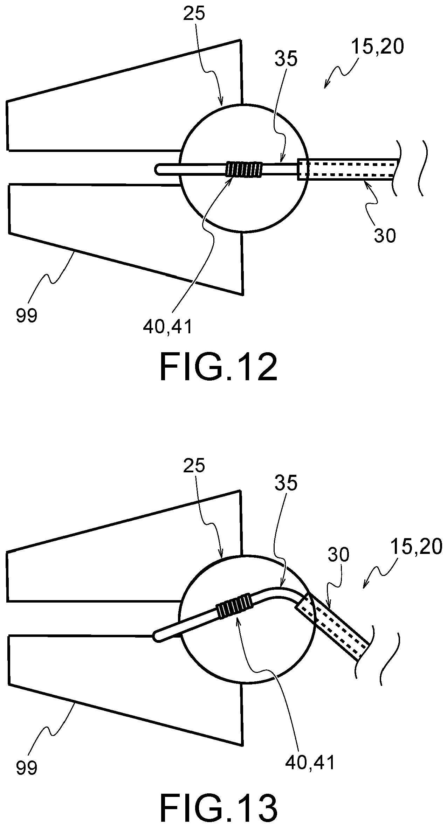

An orientation in a region close to the distal end of the catheter body 20 including the balloon 25 pressed against the pseudo-living body 99 was tested in two levels, i.e., the coaxial state shown in Fig. 12 and corresponding to Fig. 6, and the non-coaxial state shown in Fig. 13 and corresponding to Fig. 7. As the coaxial state shown in Fig. 12, the balloon 25 was pressed against the pseudo-living body 99 as a target site from the longitudinal direction LD. On the other hand, as the non-coaxial state shown in Fig. 13, the balloon 25 was pressed against the pseudo-living body 99 from a direction inclined to the longitudinal direction LD. In the non-coaxial state, the inner cylinder shaft 35 was bent at an angle between 30° and 45°. In the non-coaxial state, the balloon 25 had a shape that was not symmetrical around the outer cylinder shaft 30 and the inner cylinder shaft 35.

The agitator 75 was configured to supply and discharge a liquid, which was similar to the liquid supplied to the liquid delivery path LP and the balloon 25, to and from the liquid delivery path LP at a drive frequency of 2 Hz. A liquid supply amount from the agitator 75 to the liquid delivery path LP per supply, and a liquid discharge amount from the liquid delivery path LP to the agitator 75 per discharge were 780 mm³. A cross-sectional area of the liquid delivery path LP of the used catheter body 20 was 4376 mm². On the other hand, the length DX (see Fig. 2) from the distal end 30a of the outer cylinder shaft 30 up to the temperature sensor 45 was 150 mm.

According to the above tendency described with reference to Figs. 8 and 9, a liquid temperature fluctuation in the liquid delivery path LP is expected to take a local maximum value and a local minimum value at the same time interval as the drive cycle of the agitator 75. More specifically, when the agitator 75 discharges the liquid from the liquid delivery path LP so that the liquid is sucked from the balloon 25 into the liquid delivery path 25, a temperature of the heated high-temperature liquid is measured as a local maximum value. In addition, when the agitator 75 supplies the liquid to the liquid delivery path LP so that the liquid is ejected to the balloon 25 from the liquid delivery path LP, a temperature of a low-temperature liquid staying in the liquid delivery path LP is measured as a local minimum value. Thus, in order to understand a liquid temperature fluctuation in the liquid delivery path LP, an output of the temperature sensor 45 is preferably obtained at an interval less than the drive cycle of the agitator 75. In this experiment, since the drive frequency of the agitator 75 was 2 Hz, it was expected that a temperature fluctuation that took a local maximum value every 0.5 seconds was repeated. Thus, an output of the temperature sensor 45 was measured at a time interval sufficiently shorter than 0.5 seconds, specifically, at 10 mm second interval. Namely, a liquid temperature in the liquid delivery path LP was calculated by processing information from the temperature sensor 45 at a time interval of 1/50 of the drive cycle of the agitator 75.

The high-frequency current conduction controller 70A of the temperature control unit 70 controlled the high-frequency current conduction to the coil electrode 41 in such a manner that a temperature of the liquid around the coil electrode 41 was 70°C. A drive power was set at 150 W.

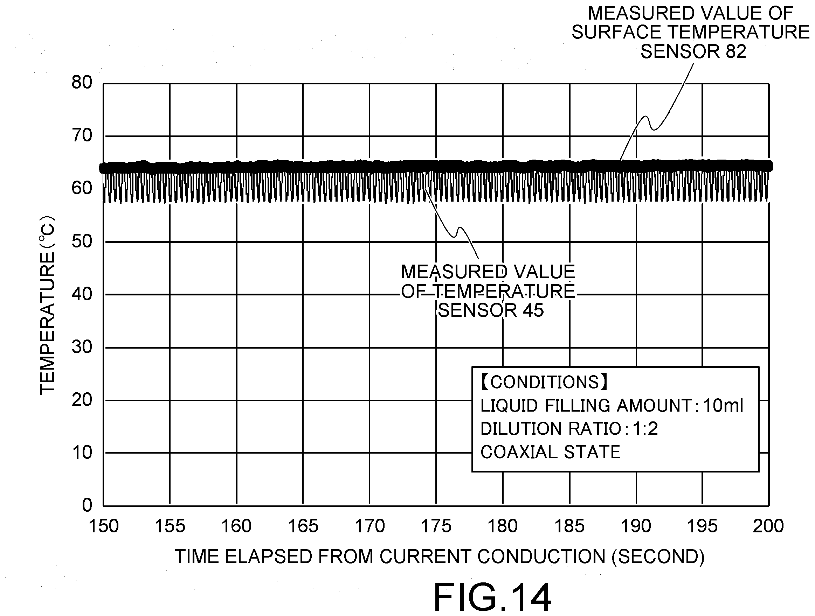

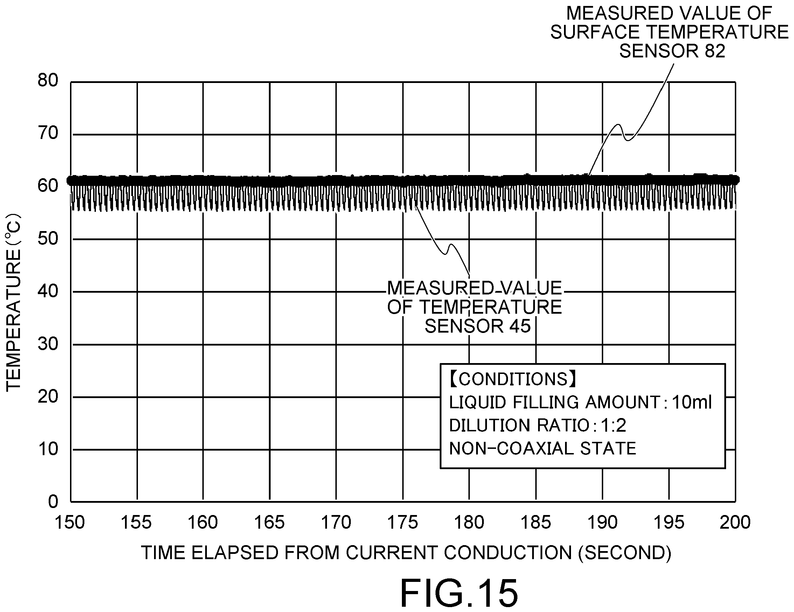

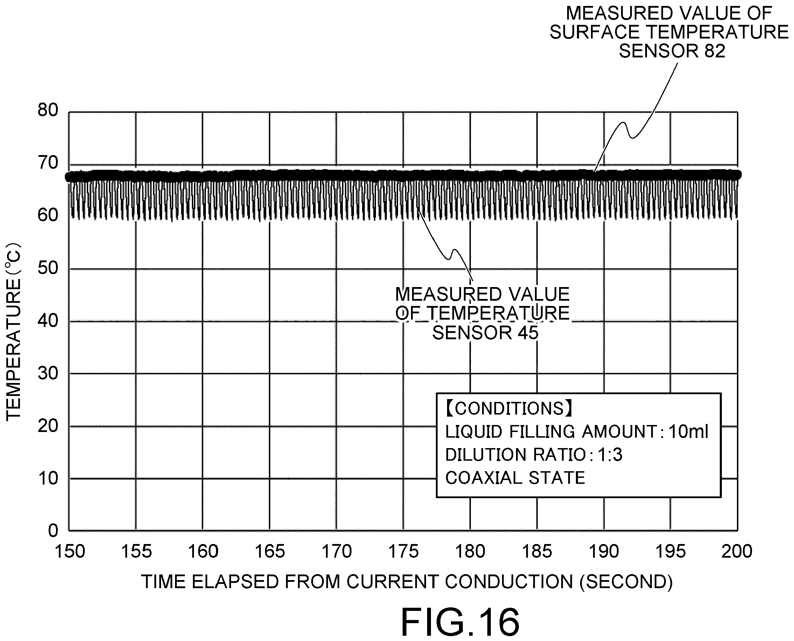

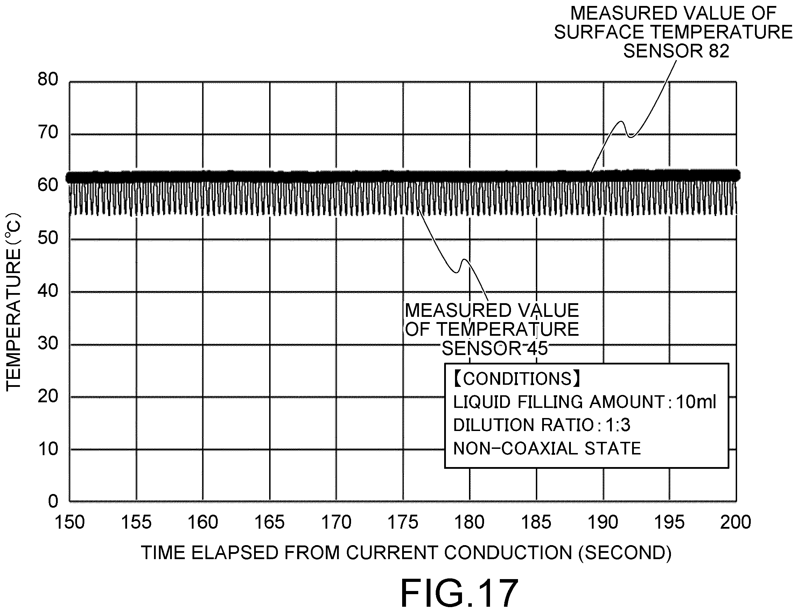

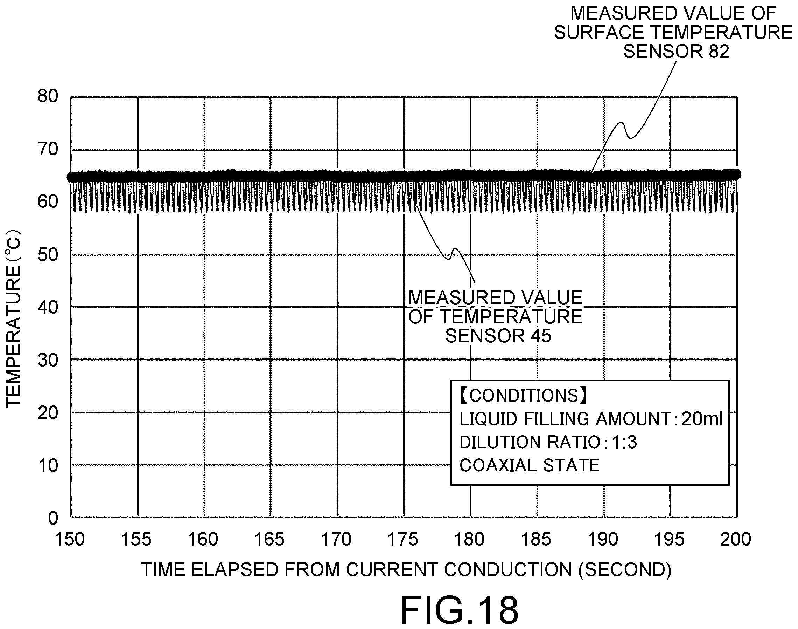

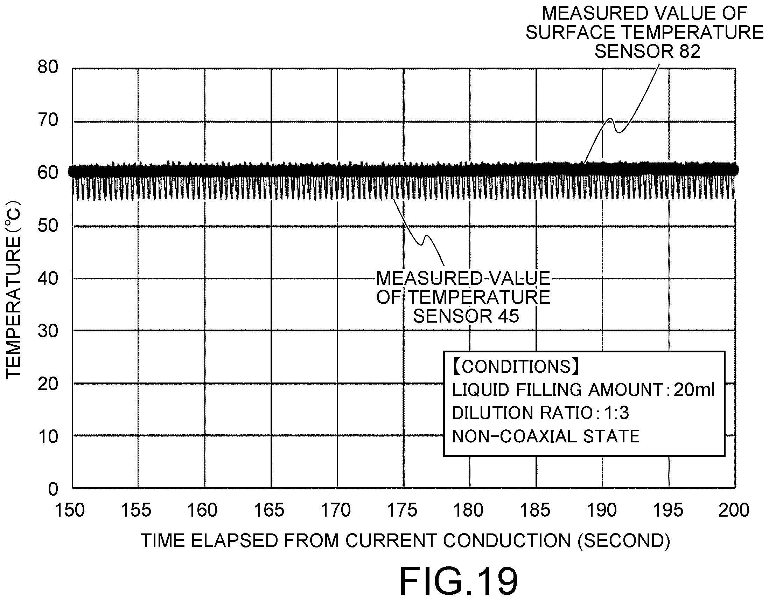

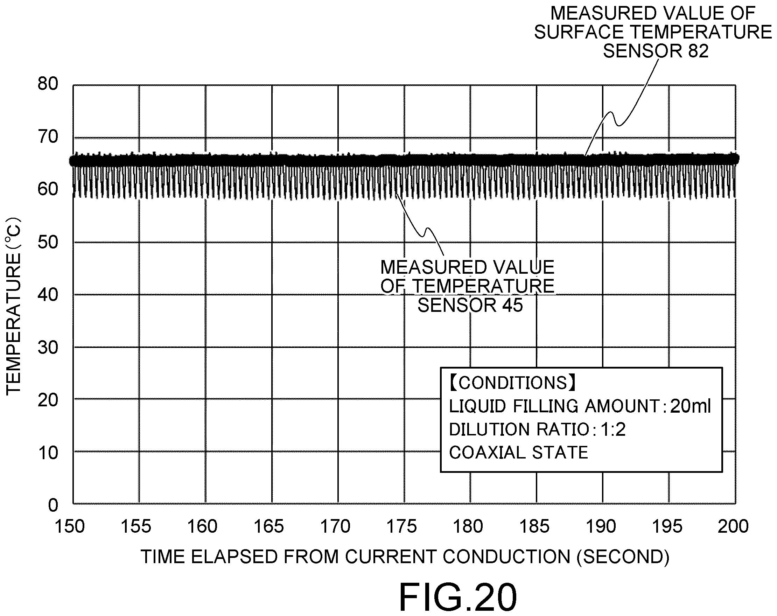

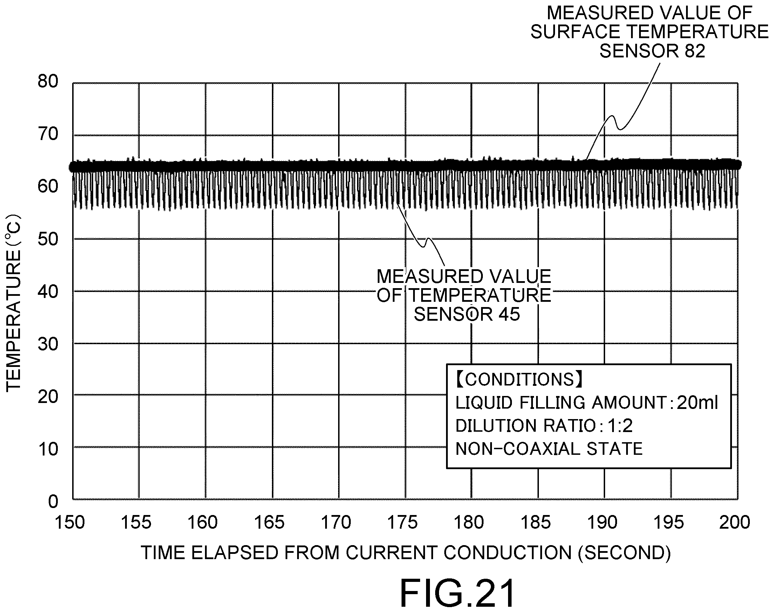

As described above, simulation tests wherein the balloon catheter system 10 was applied to the pseudo-living body 99 were performed under eight conditions in total, with two levels of liquid filling amount to the balloon 25, two levels of contrast agent dilution ratio, and two levels of pressing manner of the balloon 25. Figs. 14 to 21 are graphs showing, together with temperature values measured by the surface temperature sensor 82, temperature values measured by the temperature sensor 45 at 10 ms (mm second) interval in each experiment. In the graphs shown in Figs. 14 to 21, an axis of ordinate shows a temperature (°C) and an axis of abscissa shows a time (s) from the start of current conduction to the coil electrode 41 and the return pad 87. The graphs shown in Figs. 14 to 21 show measurement results after a time had elapsed for a surface temperature of the balloon 25 to sufficiently stabilize from the start of high-frequency current conduction, specifically, from 150 to 200 seconds after the start of current conduction.

In the results shown in Figs. 14 to 21, the liquid temperature in the liquid delivery path LP measured by the temperature sensor 45 fluctuates at about 0.5 second cycle. The temperature fluctuation cycle well conforms to a cycle of liquid supply and discharge by the agitator 75 at a drive frequency of 2 Hz. In addition, an actual measured value of a surface temperature of the balloon 25 measured by the surface temperature sensor 82 is substantially the same as local maximum values in a temperature fluctuation measured by the temperature sensor 45. From these points, the temperature calculation unit 70B of the control unit 70 can determine local maximum values of a temperature fluctuation line obtained by connecting values actually measured by the temperature sensor 45 (or an envelope connecting local maximum values) as a surface temperature of the balloon 25. Alternatively, the temperature calculation unit 70B of the temperature control unit 70 can determine first an approximate curve having a triangular wave shape from values actually measured by the temperature sensor 45, and then can determine local maximum values of this temperature fluctuation approximate curve (or an envelope connecting local maximum values) as a surface temperature of the balloon 25. A surface temperature of the balloon 25, which was estimated from the liquid temperature in the outer cylinder shaft 30 in this manner, had a significantly excellent precision. Namely, an error between the estimated temperature and the actual measured value measured by the surface temperature sensor 82 was about ±1°C.

Further, a fluctuation range of the liquid temperature in the liquid delivery path LP measured by the temperature sensor 45 was approximately 6°C to 9°C. Thus, a value obtained by the temperature calculation unit 70B of the control unit 70, which adds a specific value between 3°C or more and 5°C or less to an average value of the liquid temperatures in the liquid delivery path LP actually measured by the temperature sensor 45, can be determined as a surface temperature of the balloon 25. A surface temperature of the balloon 25, which was estimated from the liquid temperature in the outer cylinder shaft 30 in this manner, also had a significantly excellent precision. Namely, an error between the estimated temperature and the actual measured value measured by the surface temperature sensor 82 was about ±1°C.

The following finding was obtained from the experiment results shown in Figs. 14 to 20. First, the local maximum value of the liquid temperature fluctuation (or the envelope connecting the local maximum values) in the liquid delivery path LP, which was measured by the temperature sensor 45, conformed, at high precision of about ±1°C, to the actual measured value of the surface temperature of the balloon, which was measured by the surface temperature sensor 82, regardless of the state, i.e., the coaxial state or the non-coaxial state of the balloon, regardless of the amount of liquid filled to the balloon 25, and regardless of the contrast agent dilution ratio of the liquid filled to the balloon 25.

Second, a surface temperature of the balloon differs by about 5°C between the coaxial state and the non-coaxial state. Namely, when the balloon shape is symmetrical with respect to the outer cylinder shaft 30 and the inner cylinder shaft 35, the liquid in the balloon is well agitated so that a surface temperature of the balloon 25 increases. Conversely, when the shape of the balloon 25 is asymmetrical with respect to the outer cylinder shaft 30 and the inner cylinder shaft 35, it was confirmed that, since agitation of the liquid in the balloon was insufficient, a surface temperature of the balloon did not increase. This tendency was observed regardless of the filling amount of liquid into the balloon 25 and the contrast agent dilution ratio.

When the filling amount of liquid into the balloon 25 increased from 10 ml to 20 ml, a surface temperature of the balloon tended to decrease by about 2°C. However, unaffected by the filling amount of liquid into the balloon 25, the local maximum values of the liquid temperature fluctuation (or the envelope connecting the local maximum values) in the liquid delivery path LP, which was measured by the temperature sensor 45, conformed, at high precision of about ±1°C, to the actual measured value of the surface temperature of the balloon, which was measured by the surface temperature sensor 82. Similarly, when the contrast agent dilution rate was either 1:2 or 1:3, i.e., unaffected by the contrast agent dilution ratio, the local maximum values of the liquid temperature fluctuation (or the envelope connecting the local maximum values) in the liquid delivery path LP, which was measured by the temperature sensor 45, conformed, at high precision of about ±1°C, to the actual measured value of the surface temperature of the balloon, which was measured by the surface temperature sensor 82.

The above experiment results shown in Figs. 14 to 21 can be summarized as follows. In accordance with the liquid supply from the agitator 75 to the liquid delivery path LP, the liquid temperature measured by the temperature sensor 45 took local minimum values. In addition, in accordance with the liquid discharge from the liquid delivery path LP to the agitator 75, the liquid temperature measured by the temperature sensor 45 took local maximum values. By continuously monitoring peak values (local maximum values) of the liquid temperature detected by the temperature sensor 45, a surface temperature of the balloon could be highly precisely understood. Specifically, both when the balloon shape was in the coaxial state and in the non-coaxial state, unaffected by the filling amount of liquid into the balloon 25 and the contrast agent dilution ratio, the local maximum values of the liquid temperature fluctuation (or the envelope connecting the maxima) in the liquid delivery path LP, which was measured by the temperature sensor 45, conformed, at high precision of about ±1°C, to the actual measured value of the surface temperature of the balloon, which was measured by the surface temperature sensor 82. Thus, a surface temperature of the balloon 25, which is most important in the ablation therapy, could be precisely detected, displayed and used in the control.

The experiment results shown in Figs. 14 to 21 can be summarized as followed. The liquid temperature fluctuation in the liquid delivery path LP took the local maximum values and the local minimum values at the same cycle as the drive cycle of the agitator 75. In accordance with the liquid supply from the agitator 75 to the liquid delivery path LP, the liquid temperature measured by the temperature sensor 45 took the local minimum values. In addition, in accordance with the liquid discharge from the liquid delivery path LP to the agitator 75, the liquid temperature measured by the temperature sensor 45 took the local maximum values. By continuously monitoring peak values (local maximum values) of the liquid temperature detected by the temperature sensor 45, a surface temperature of the balloon could be highly precisely understood. Specifically, both when the balloon shape was in the coaxial state and in the non-coaxial state, unaffected by the filling amount of liquid into the balloon 25 and the contrast agent dilution ratio, the local maximum values of the liquid temperature fluctuation (or the envelope connecting the local maximum values) in the liquid delivery path LP, which was measured by the temperature sensor 45, conformed, at high precision of about ±1°C, to the actual measured value of the surface temperature of the balloon, which was measured by the surface temperature sensor 82. Thus, a surface temperature of the balloon 25, which is most important in the ablation therapy, could be precisely detected, displayed and used in the control.

The aforementioned experiment results show that, in order to understand a liquid temperature fluctuation in the liquid delivery path LP, it is preferable that a detection result (information acquired by the temperature sensor 45) is acquired at a time interval less than the drive cycle of the agitator 75 to determine the liquid temperature. In addition, it is more preferable that a detection result is acquired from the temperature sensor 45 at a time interval less than half of the drive cycle of the agitator 75. In this case, the temperature can be detected at least once while the liquid temperature in the liquid delivery path LP is decreasing, and the temperature can be detected at least once while the liquid temperature in the liquid delivery path LP is increasing. Thus, it is advantageous to acquire an output from the temperature sensor 45 at a time interval less than half the drive cycle of the agitator 75, in order to understand an average temperature of liquid in the liquid delivery path LP.

Similarly, it is preferable that a detection result is acquired at a time interval less than 1/4 of the drive cycle of the agitator 75 to determine the liquid temperature. In this case, the temperature can be detected at least twice while the liquid temperature in the liquid delivery path LP is decreasing, and the temperature can be detected at least twice while the liquid temperature in the liquid delivery path LP is increasing. Thus, it is advantageous to acquire an output from the temperature sensor 45 at a time interval less than 1/4 of the drive cycle of the agitator 75, in order to understand a temperature fluctuation profile of liquid in the liquid delivery path LP.

When the local maximum values in the liquid temperature fluctuation in the liquid delivery path LP which serves as a reference of a surface temperature of the balloon 25 are detected, the shorter the time interval at which a detection result is acquired from the temperature 45 to determine the liquid temperature is, the better the detection precision is. From this point, a time interval at which an output of the temperature sensor 45 is obtained to determine a temperature liquid is preferably less than 1/5 of the drive cycle of the agitator 75, more preferably less than 1/8 of the drive cycle of the agitator 75, further preferably less than 1/10 of the drive cycle of the agitator 75. It is furthermore preferable to continuously determine a liquid temperature.

A "local maximum value" in a temperature fluctuation of liquid, which is determined from information acquired by the temperature sensor 45, means a temperature value (°C) at the time when a temperature changes from an increase to a decrease in a temperature fluctuation with time. A "local maximum value" is not limited to a strict mathematical meaning and may be determined by a polygonal line connecting temperature values measured at a predetermined time interval with straight lines, or may be determined by a continuously linear fluctuation (e.g., an approximate curve having a triangular wave shape) obtained by approximating temperature values measured at a predetermined time interval with curves. Similarly, a "local minimum value" in a temperature fluctuation of liquid, which is determined from information acquired by the temperature sensor 45, means a temperature value (°C) at the time when a temperature changes from a decrease to an increase in a temperature fluctuation with time. A "local minimum value" is not limited to a strict mathematical meaning and may be determined by a polygonal line connecting temperature values measured at a predetermined time interval with straight lines, or may be determined by a continuously linear fluctuation (e.g., an approximate curve having a triangular wave shape) obtained by approximating temperature values measured at a predetermined time interval with curves. Further, an envelope connecting local maximum values in a temperature fluctuation used in this specification is not limited to a strict mathematical meaning and means a polygonal or continuously linear line sequentially connecting local maxima values in the temperature fluctuation.

Some specific examples included in the aforementioned embodiment are described hereunder. In the below description of the specific examples and the drawings used in the below description of the specific examples, the same or similar numerals are given to the same parts or parts having similar functions, and the repeated description thereof may be omitted.

### <First Specific Example>

### (Manufacture of ablation catheter system with balloon)

A polyurethane balloon 25 having a diameter of 30 mm and a thickness of 20 µm was manufactured by blow molding using a polyurethane tube. A polyurethane tube which had an external diameter of 3.6 mm, an internal diameter of 3.0 mm, and a length of 1000 mm, was molded and used as the outer cylinder shaft 30. A polyamide tube, which had an external diameter of 1.6 mm, an internal diameter of 1.2 mm, and a length of 1100 mm was molded and used as the inner cylinder shaft 35. The handle 50 was connected to the rear ends (proximal ends) of the outer cylinder shaft 30 and the inner cylinder shaft 35.

A copper wire having a diameter of 0.26 mm and a length of 1700 mm, which was coated with an electrically insulating film made of perfluoroalkoxy alkane was used as the wiring 42. The electrically insulating film on the wiring 42 was peeled by 200 mm, and the wiring 42 from which the film had been peeled was wound like a coil around the inner cylinder shaft 35, starting from a position at 25 mm from the distant end (distal end) of the inner cylinder shaft 35 to obtain the coil electrode 41 for high-frequency current conduction. Polyurethane tubes were fixed by heat welding at positions on the inner cylinder shaft 35 adjacent to the coil electrode 41 from both sides in the longitudinal direction LD. The polyurethane tubes were provided in order to prevent displacement of the coil electrode 41 on the inner cylinder shaft 35.

The temperature sensor 45 was installed in the inner surface of the outer cylinder shaft 30 at a position 10 mm from the distant end (distal end) thereof. A heat shrinkable tube serving as the fixing means 48 was disposed on the inner cylinder shaft 35. The heat shrinkable tube was heated on the inner cylinder shaft 35 such that the lead wire 47 of the temperature sensor 45 passed through the heat shrinkable tube. The lead wire 47 was fixed on the inner cylinder shaft 35 by the heat shrinkable tube shrunk by heating. The thermosensitive part 46 of the temperature sensor 45, which was attached to the inner cylinder shaft 35 by means of the fixing means 48, was spaced apart from the outer cylinder shaft 30 and the inner cylinder shaft 35.

The distant side (distal side) portion of the inner cylinder shaft 35 was inserted to the balloon 25, and the rear end portion (proximal end portion) of the balloon was fixed on the distant end portion (distal end portion) of the outer cylinder shaft 30 by heat welding. In addition, the distant end portion (distal end portion) of the balloon 25 was fixed on the inner cylinder shaft 35 by heat welding.

The rear end side of the wiring 42 electrically connected to the coil electrode 41 was electrically connected to the high-frequency current conduction controller 70A of the temperature control unit 70, by passing the wiring 42 through the liquid delivery path LP between the outer cylinder shaft 30 and the inner cylinder shaft 35 and the inside of the handle 50. Similarly, the lead wire 47 of the temperature sensor 45 was electrically connected to the temperature calculation unit 70B of the control unit 70 by passing the lead wire 47 through the liquid delivery path LP and the inside of the handle 50.

The valve 58 was attached to the branch of the handle 50 through the extension tube 57. A three-way stopper cock was used as the valve 58. Further, the valve 58 and the agitator 75 were connected through the extension tube 57. Thus, a way through which vibrations given from the agitator 75 to a liquid was transmitted to a liquid inside the balloon 25 through the extension tube 57, the handle 50 and the liquid delivery path LP to agitate the liquid was formed.

### (Control System and Control Method)

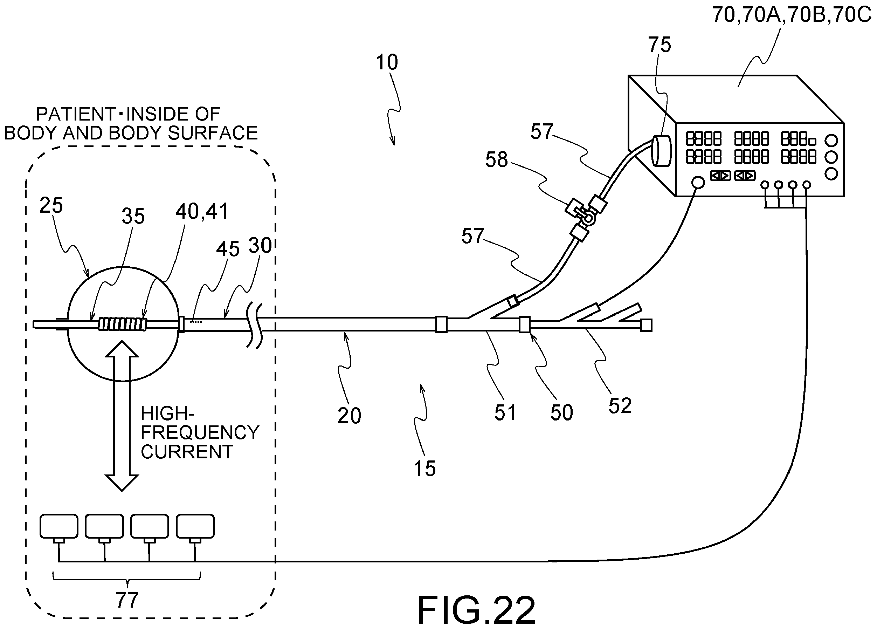

In the aforementioned experiments using the balloon catheter system of Fig. 10, information (electric potential) acquired by the electrode temperature sensor 81 was introduced to the temperature calculation unit 70B and the high-frequency current conduction controller 70A of the control unit 70. Based on the fact that a temperature difference between a surface temperature of the balloon 25 and an ambient temperature of the coil electrode 41 was about 5°C, application of a high-frequency voltage to the coil electrode 41 was adjusted to adjust an output of the coil electrode 41, in such a manner that a liquid temperature around the coil electrode 41, which was determined from information acquired by the electrode temperature sensor 81, became 70°C, in order to control a surface temperature of the balloon 25 to 65°C. On the other hand, in the balloon catheter system 10 in the first specific example, as shown in Fig. 22, information (electric potential) acquired by the temperature sensor 45 was introduced to the temperature calculation unit 70B of the control unit 70. Application of a high-frequency voltage from the high-frequency current conduction controller 70A to the coil electrode 41 was adjusted to control an output of the coil electrode 41, in such a manner that a surface temperature of the balloon 25, which was calculated by the temperature calculation unit 70B based on the information acquired by the temperature sensor 45, became 65°C. As described above, a local maximum value (peak value) of a temperature fluctuation of liquid in the liquid delivery path LP, which was determined from the information acquired by the temperature sensor 45, was outputted as a surface temperature of the balloon 25. In the balloon catheter system 10 shown in Fig. 22, the high-frequency current conduction controller 70A, the temperature calculation unit 70B, and the agitator controller 70C are accommodated in one housing. The high-frequency current conduction controller 70A, the temperature calculation unit 70B, and the agitator controller 70C may share at least one structure (hardware).

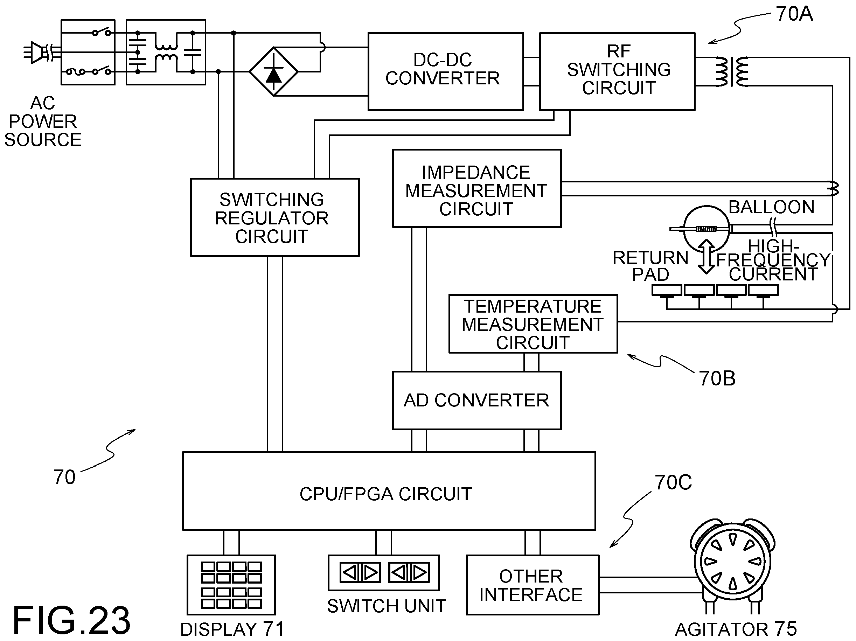

A specific control method of the system according to the first specific example is described using a block diagram shown in Fig. 23. An AC power source input was inputted via a power source line filter to a full-wave rectifier circuit, while avoiding emission of power source noise by a high-frequency power circuit. On the other hand, in order to obtain a stabilized DC power supply for a logic circuit including CPU and FPGA, a switching regulator was incorporated.

A current outputted from the full-wave rectifier circuit was converted to a direct current having a further higher electric potential (usually 400 v to 500 v) by a DC-DC converter in a next state, and was further converted to a square wave of alternating current (usually a frequency of 200 KHz to 3 MHz) by an RF switching circuit that was a high-speed chopper circuit using MOSFET (not shown). This conversion to the alternating current made possible that, even when a current was caused to flow through a human body between the coil electrode 41 of the heating component 40 and the current electrode 77, the current was harmless to human.

Information as a detection result by the temperature sensor 45 was imported to a temperature measurement circuit through the lead wire 47 passing through the catheter body 20. The information was further introduced through an AD conversion circuit to a logic circuit including CPU and FPGA that performed signal processing and control. An envelope connecting local maximum values and local minimum values in a temperature fluctuation of liquid in the liquid delivery path LP, which were determined from the information acquired by the temperature sensor 45, was extracted. Thus, a surface temperature of the balloon 25 could be precisely detected and monitored. In addition, an RF switching circuit was controlled such that a surface temperature of the balloon 25 to be monitored had always a desired temperature.

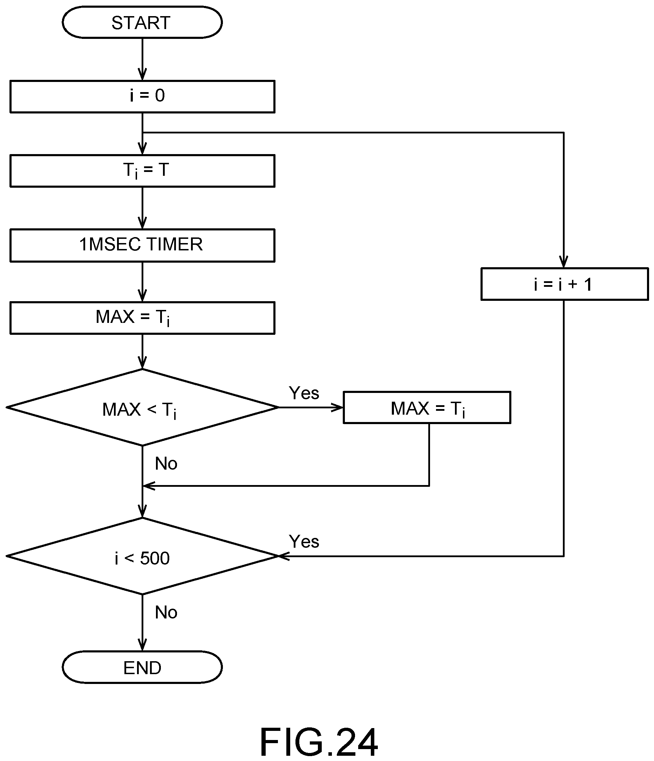

There are various methods for extracting an envelope connecting local maxima values in a temperature fluctuation of liquid in the liquid delivery path LP, which are determined from the information acquired by the temperature sensor 45. The first specific example used a method shown by a flowchart of Fig. 24. Namely, a n output from the temperature sensor 45 was converted at 1 mm second interval to a digital temperature signal by an AD conversion circuit. A largest value (denoted as MAX) of the output (denoted as T) converted at 1 mm second interval to the digital signal was recorded at a vibration cycle (500 ms interval) of the agitator 75 and used as a temperature control signal. Namely, the largest value used in the temperature control signal in the process shown in Fig. 24 was local maximum values in a polygonal line temperature fluctuation obtained by connecting, with straight lines, liquid temperature values in the liquid delivery path LP which were regularly determined by the information from the temperature sensor 45. This maximum was used in the control as a surface temperature of the balloon 25.

An employed fail-safe mechanism constantly monitored a current flowing between the coil electrode 41 and the return pad 77 and imported the current to the logic circuit including CPU and FPGA through an impedance measurement circuit and the AD conversion circuit. Thus, in the event of an unexpected situation occurring in the human body and the therapy upon application of a high-frequency current, the fail-safe mechanism could promptly turn off the high-frequency current.

In order to make uniform a temperature in the balloon, an operation of the agitator 75 that vibrated and agitated the liquid through the liquid delivery path LP, setting of the system, and various interface between the system and an operator (physician) were performed by a signal from the logic circuit including CPU and FPGA.

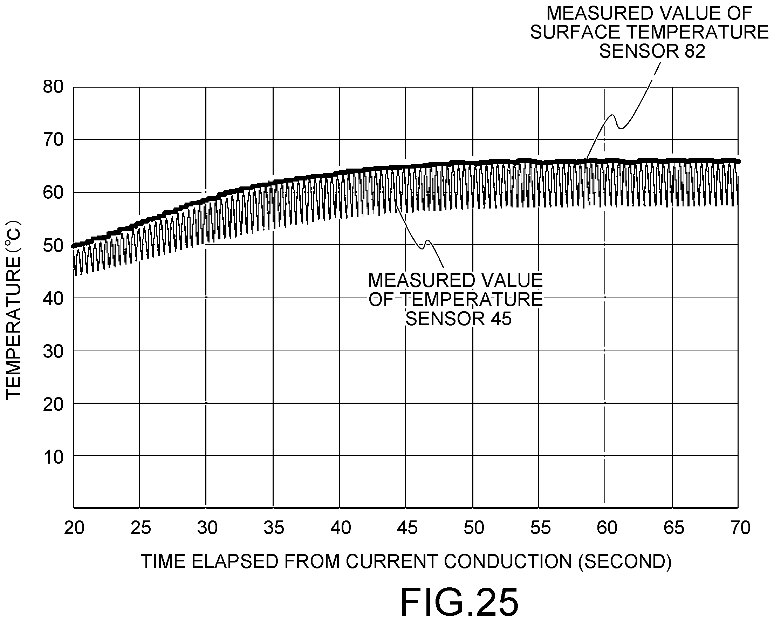

The experiment shown in Fig. 10 was conducted using the balloon catheter system 10 according to the aforementioned first specific example. The pseudo-living body 99, the water tank 85, the water-tank agitator 86, the return pad 87, and the surface temperature sensor 82 were the same as those in the aforementioned experiment. On the other hand, high-frequency current conduction to the coil electrode 41 and the return pad 87 was controlled without using the electrode temperature sensor 81, in such a manner that a surface temperature of the balloon 25 estimated from a detection result of the temperature sensor 45 was 66.0°C. As other conditions of the high-frequency current conduction, a frequency was set to 1.8 MHz, and a high-frequency power to be applied was set to 150 W. A graph of Fig. 25 shows a fluctuation of a liquid temperature in the liquid delivery path LP, which was determined from the information acquired by the temperature sensor 45, and a fluctuation of a surface temperature of the balloon, which was actually measured by the surface temperature sensor 82. As shown in Fig. 25, the local maximum values in the fluctuation of the liquid temperature in the liquid delivery path LP, which was determined from the information acquired by the temperature sensor 45, highly precisely conformed to the actual temperature of the surface of the balloon 25. Thus, the control of high-frequency current conduction while controlling a surface temperature of the balloon 25 makes it possible that a surface temperature of the balloon 25 has a target value.

### <Second Specific Example>

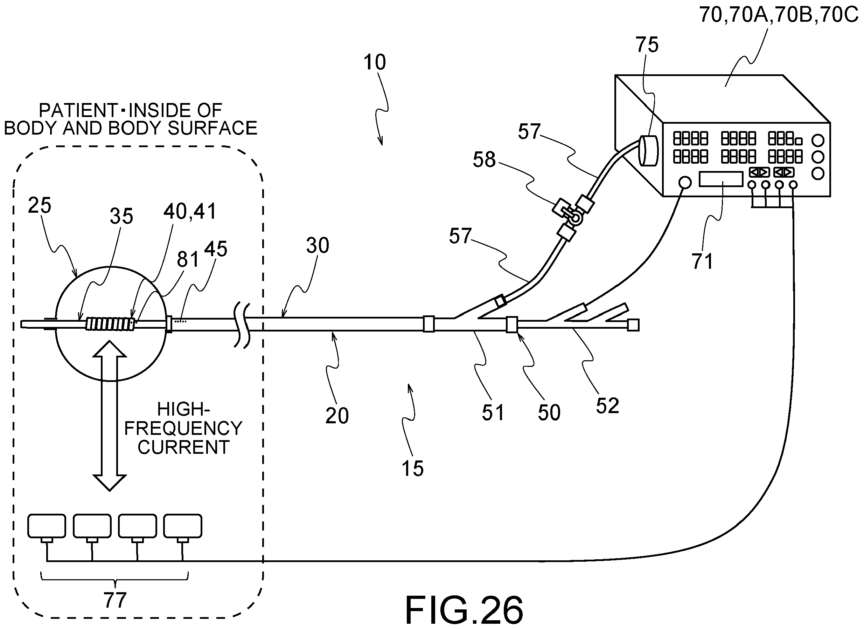

Next, Fig. 26 shows the balloon catheter system 10 of a second specific example. The balloon catheter system 10 of the second specific example had the electrode temperature sensor 81 provided on the inner cylinder shaft 35. The electrode temperature sensor 81 was a thermocouple having, as one electrode, the coil electrode 41 provided on the inner cylinder shaft 45, and, as the other electrode, a conductive wiring electrically connected to the coil electrode 41, the conductive wiring being made of a material different from that of the coil electrode 41. In the balloon catheter system 10, the high-frequency current conduction controller 70A of the control unit 70 controls high-frequency current conduction to the coil electrode based on temperature information acquired by the electrode temperature sensor 81. The balloon catheter system 10 also had the aforementioned temperature sensor 45 disposed in the liquid delivery path LP. Thus, an operator (physician) could understand a surface temperature of the balloon 25 determined from the information acquired by the temperature sensor 45 as described above. A determined surface temperature of the balloon 25 was displayed on the display 71 of the control unit 70 so that the operator could understand the surface temperature of the balloon during the operation. The display 71 of the control unit 70 also displayed a liquid temperature around the heating component 40, which was determined from a detection result of the electrode temperature sensor 81.

The balloon catheter system 10 of the second specific example differed from the aforementioned balloon catheter system 10 of the first specific example in that it had two types of temperature sensors, i.e., the electrode temperature sensor 81 and the temperature sensor 45. In the second specific example, a surface temperature of the balloon 25, which is determined as described above from the information acquired by the temperature sensor 45, can be displayed on the display 71 of the control unit 70. The second specific example employed a digital temperature indicator as means for displaying a surface temperature of the balloon 25. On the other hand, control of an output from the heating component 40 can be performed based on a temperature value determined by the electrode temperature sensor 81. Alternatively, control of an output from the heating component 40 may be performed based on a temperature value determined by one sensor selected from the electrode temperature sensor 81 and the temperature sensor 45.

Since the balloon catheter system 10 of the second specific example controls a liquid temperature around the coil electrode 41 serving as the heating component 40 in such a manner that the liquid temperature has a desired set temperature, an output of the heating component 40 serving as heating means can be directly controlled. Thus, unlike the first specific example, it is not necessary to obtain a local maximum value and an envelope of local maximum values of a liquid temperature fluctuation determined by an output of the temperature sensor and to control the ablation catheter system by the signal based on the determination. As a result, the balloon catheter system 10 can be controlled while eliminating the influence on the control of the balloon catheter system 10, which is caused by delay or the like because of a signal processing time in the temperature detection circuit is eliminated.

In the balloon catheter system 10 of the second specific example, local maximum values and an envelope of local maximum values of a liquid temperature fluctuation determined by an output of the temperature sensor 45 is obtained, and the display 71 displays it in a digital or analog waveform, whereby a physician can be informed of a balloon surface temperature in real time.

### <Third Specific Example>

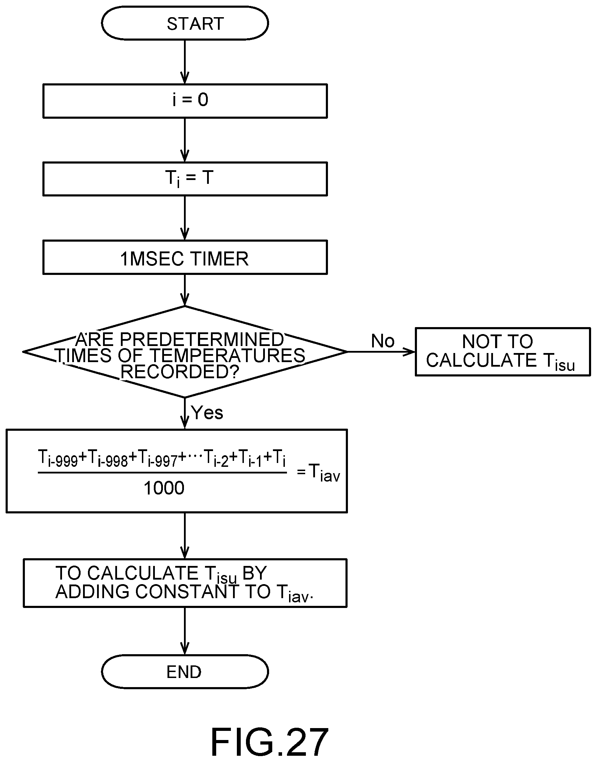

Further, Fig. 27 shows the balloon catheter system 10 of a third specific example. In the balloon catheter system 10 of the third specific example, similarly to the first specific example, information acquired by the temperature sensor 45 was introduced to the temperature calculation unit 70B of the control unit 70, and an output of the coil electrode 41 was controlled by controlling high-frequency current conduction to the coil electrode 41 based on a liquid temperature determined by the high-frequency current conduction controller 70A from the information acquired by the temperature sensor 45. At this time, similarly to the first specific example, local maximum values and an envelope connecting local maximum values of a liquid temperature fluctuation determined from the output of the temperature sensor 45 could be detected at sufficiently high speed, and a surface temperature of the balloon 25 could be highly precisely determined from the temperature sensor 45.

On the other hand, similarly to the second specific example, the third specific example has the electrode temperature sensor 81 provided on the inner cylinder shaft 35. The temperature calculation unit 70B of the control unit 70 calculates a temperature of a liquid around the coil electrode 41 from information acquired by the electrode temperature sensor 81. This allows the control unit 70 to monitor occurrence of a situation where a liquid temperature determined based on the information from the electrode temperature sensor 81 exceeds a boiling point of the liquid. When the liquid in the balloon 25 exceeds the boiling point, undesirable bubbles are generated in the balloon 25. The high-frequency current conduction controller 70A of the control unit 70 controlled an output of the coil electrode 41 by adjusting high-frequency current conduction to the coil electrode 41 in order not to generate bubbles in the balloon 25.

### <Fourth Specific Example>

A fourth specific example had the same devices and electric circuit structure as the balloon catheter system 10 according to the first specific example. However, the fourth specific example differed from the first specific example in a method of determining a surface temperature of the balloon 25 from information acquired by the temperature sensor 45. In the fourth specific example, an average value of liquid temperatures in the liquid delivery path LP determined from the information acquired by the temperature sensor 45 was obtained, and a value obtained by adding an offset value to the average value was determined as a surface temperature of the balloon 25.

In the aforementioned experiment result by the inventors, a fluctuation range of the liquid temperature in the liquid delivery path LP was approximately 6°C to 9°C. Thus, a value obtained by adding a specific value between 3°C or more and 5°C or less to an average value of the liquid temperatures in the liquid delivery path LP, which are actually measured by the temperature sensor 45, can be determined as a surface temperature of the balloon 25. A surface temperature of the balloon 25, which was estimated from the average value of the liquid temperatures in the outer cylinder shaft 30 in this manner, had a significantly excellent precision. Namely, an error between the estimated temperature and the actual measured value measured by the surface temperature sensor 82 was about ±1°C. In the fourth specific example, an output from the coil electrode 41 was controlled by adjusting high-frequency current conduction to the coil electrode 41 based on the thus determined surface temperature of the balloon 25.

Fig. 27 shows a flowchart along which an average value of liquid temperatures in the liquid delivery path LP, which is calculated by information from the temperature sensor 45 used in the fourth specific example. An output from the temperature sensor 45 was converted at 1 mm second interval to a digital temperature signal by an AD conversion circuit. The converted output was imported as a temperature sensor output in the shaft (T). A moving average of the outputs (T) of the temperature sensor 45 at 1 second interval was denoted as Tav. Since an output average value of the temperature sensor 45 had an offset of about -4°C with respect to a local maximum value of outputs of the temperature sensor 45, a value obtained by adding 4°C to Tav was used as a surface temperature Tsu of the balloon. Other control manners were similar to the first specific example. The flowchart method used herein is nothing more than an example for obtaining an average value of liquid temperatures in the liquid delivery path LP measured by the temperature sensor 45.

In the aforementioned embodiment, the balloon catheter 15 has: the balloon 25; the outer cylinder shaft 30 connected to the proximal end 25b of the balloon 25; the inner cylinder shaft 35 passing through the outer cylinder shaft 30 to extend into the balloon 25 to be connected to the distal end 25a of the balloon 25, the inner cylinder shaft 35 forming between it and the outer cylinder shaft 30 a liquid delivery path LP in communication with an inside of the balloon 25; and the heating component 40 disposed in the balloon 25 for heating a liquid in the balloon 25. In such a balloon catheter 15, a surface temperature of the balloon 25 and a temperature in the liquid delivery path LP in the outer cylinder shaft 30 that sucks the liquid in the balloon 25 have a strong correlation. In particular, a liquid temperature in a region of the liquid delivery path LP close to the distal end 30a of the outer cylinder shaft 30 has a strong correlation to a surface temperature of the balloon 25. In the aforementioned embodiment, the temperature sensor 45 that acquires information on a temperature in the liquid delivery path LP is provided between the outer cylinder shaft 30 and the inner cylinder shaft 35. Since a surface temperature of the balloon 25 has a strong correlation to a liquid temperature in the liquid delivery path LP into which a liquid in the balloon 25 flows, a surface temperature of the balloon 25 can be highly precisely detected based on information acquired by the temperature sensor 45 in the liquid delivery path LP. This can significantly improve an ablation therapy effect. In addition, as compared with the balloon 25 which largely deforms by inflation and deflation, the outer cylinder shaft 30 and the inner cylinder shaft 35 can stably hold the temperature sensor 45 in the liquid delivery path LP.

In one specific example of the aforementioned embodiment, a length along the longitudinal direction LD from the distal end 30a of the outer cylinder shaft 30 up to the temperature sensor 45 is between 5 mm or more and 150 mm or less. This specific example allows that, when liquid injection from the liquid delivery path LP into the balloon 25 and liquid suction from the balloon 25 to the liquid delivery path LP are repeated, the liquid in the balloon 25, in particular, the liquid close to the surface of the balloon 25 can be drawn into the outer cylinder shaft 30, and a surface temperature of the balloon 25 can be highly precisely evaluated by the temperature sensor 45 in the outer cylinder shaft 30.

In one specific example of the aforementioned embodiment, the temperature sensor 45 is attached to the inner cylinder shaft 35, the inner cylinder shaft is relatively movable with respect to the outer cylinder shaft 30, and when the inner cylinder shaft 35 is relatively moved to the distal side from the outer cylinder shaft 35 so that the balloon 25 is stretched, the temperature sensor 45 is positioned in the liquid delivery path LP between the outer cylinder shaft 30 and the inner cylinder shaft 35. This specific example allows the temperature sensor 45 to be positioned between the outer cylinder shaft 30 and the inner cylinder shaft 35, regardless of a relative position of the inner cylinder shaft 35 with respect to the outer cylinder shaft 30. Thus, the temperature sensor 45 can be stably protected by the outer cylinder shaft 30, regardless of a relative position of the inner cylinder shaft 35 with respect to the outer cylinder shaft 30.

In one specific example of the aforementioned embodiment, the temperature sensor 45 may be attached to the outer cylinder shaft 30. This specific example allows the temperature sensor 45 to be positioned in the outer cylinder shaft 30. Thus, the temperature sensor 45 can be stably protected by the outer cylinder shaft 30, regardless of a relative position of the inner cylinder shaft 35 with respect to the outer cylinder shaft 30.

In one specific example of the aforementioned embodiment, the temperature sensor 45 includes the thermosensitive part 46, and the lead wire 47 connected to the thermosensitive part 46, the lead wire 47 is fixed on the inner cylinder shaft 35 or the outer cylinder shaft 30, while the thermosensitive part 46 is apart from the inner cylinder shaft 35 and the outer cylinder shaft 30. This specific example allows the temperature sensor 45 to be attached to the inner cylinder shaft 35 or the outer cylinder shaft 30 through the lead wire 47. On the other hand, since the thermosensitive part 46 is maintained not in contact with the inner cylinder shaft 35 and the outer cylinder shaft 30 having a large heat capacity, a liquid temperature in the liquid delivery path LP can be highly precisely and promptly understood.

In one specific example of the aforementioned embodiment, the control unit 70 first determines a temperature fluctuation of the liquid in the liquid delivery path LP from information acquired by the temperature sensor 45, and then determines a surface temperature of the balloon 25 based on the determined temperature fluctuation. This specific example allows a surface temperature of the balloon 25 to be highly precisely detected, as verified by the aforementioned experiment. Thus, an ablation therapy effect can be improved.

In one specific example of the aforementioned embodiment, the control unit 70 first determines a temperature fluctuation of the liquid in the liquid delivery path LP from information acquired by the temperature sensor 45, and then determines a local maximum value of the determined temperature fluctuation as a surface temperature of the balloon 25. This specific example allows a surface temperature of the balloon 25 to be highly precisely detected, as verified by the aforementioned experiment. Thus, an ablation therapy effect can be improved.

In one specific example of the aforementioned embodiment, the balloon catheter system 10 has the agitator 75 that repeats supply of liquid to the liquid delivery path LP and discharge of liquid from the liquid delivery path LP at a regular cycle. The control unit 70 acquires and process information from the temperature sensor 45 at a time interval less than the regular cycle. This specific example allows a surface temperature of the balloon 25 to be highly precisely detected, as verified by the aforementioned experiment. Thus, an ablation therapy effect can be improved.

In one specific example of the aforementioned embodiment, the balloon catheter system 10 has the agitator 75 that repeats supply of liquid to the liquid delivery path LP and discharge of liquid from the liquid delivery path LP at a regular cycle. A length [mm] along the longitudinal direction LD from the distal end 30a of the outer cylinder shaft 30 up to the temperature sensor 45 is a value equal to or less than a value obtained by dividing a predetermined amount [mm³] by a cross-sectional area [mm²] of the liquid delivery path. This specific example allows, when liquid injection into the balloon 25 through the liquid delivery path LP and liquid suction from the balloon therethrough are repeated, the liquid close to the surface of the balloon 25 can be drawn to around the temperature sensor 45 in the outer cylinder shaft 30. Thus, a surface temperature of the balloon 25 can be highly precisely evaluated by the temperature sensor 45 in the outer cylinder shaft 30.

In one specific example of the aforementioned embodiment, the wiring 42 electrically connected to the heating component 40 and the control unit 70 is provided, and the temperature sensor 45 includes the lead wire 47 electrically connected to the control unit 70. The inner cylinder shaft 35 is relatively movable with respect to the outer cylinder shaft 30. Both the wiring 42 and the lead wire 47 are attached to one and the same of the outer cylinder shaft 30 and the inner cylinder shaft 35 to extend in the liquid delivery path LP. This specific example can effectively prevent entanglement of the wiring 42 and the lead wire 47 both of which extend inside the liquid delivery path LP, when the outer cylinder shaft 30 and the inner cylinder shaft 35 are moved relatively to each other. This allows stable adjustment of a liquid temperature in the balloon by means of the heating component 40, and stable understanding of a surface temperature of the balloon 25.

In one specific example of the aforementioned embodiment, the balloon catheter 15 has: the balloon 25; the outer cylinder shaft 30 connected to the proximal end 25b of the balloon 25; the inner cylinder shaft 35 passing through the outer cylinder shaft 30 to extend into the balloon 25 to be connected to the distal end 25a of the balloon 25, the inner cylinder shaft 35 forming between it and the outer cylinder shaft 30 the liquid delivery path LP in communication with the balloon 25; and the coil electrode 41 disposed in the balloon 25 for heating a liquid in the balloon 25 by applying a high-frequency current to the liquid during high-frequency voltage supply. This embodiment allows the liquid to be heated by applying the high-frequency current to the liquid. On the other hand, since the temperature sensor 45 is shielded from the high-frequency current, a temperature of the liquid in the balloon 25 can be highly precisely detected, thereby improving an ablation therapy effect.

Although one embodiment has been described based on several example, these examples are not intended to limit one embodiment. The aforementioned embodiment can be implemented in various other examples, and can be variously omitted, replaced, modified, added, etc. without departing from the its gist.

For example, in the aforementioned embodiment, one liquid delivery path LP is provided between the outer cylinder shaft 30 and the inner cylinder shaft 35, the liquid is supplied into the balloon 25 though the one liquid delivery path LP, and the liquid is discharged from the balloon 25 therethrough. However, the present invention is not limited to the example, and two or more liquid delivery paths LP may be provided between the outer cylinder shaft 30 and the inner cylinder shaft 35. In this modification example, two or more liquid delivery paths LP may include a supply liquid delivery path for supplying a liquid into the balloon 25, and a discharge liquid delivery path for discharging a liquid from the balloon 25. Also in this modification example, a surface temperature of the balloon 25 can be highly precisely understood by the temperature sensor 45 disposed in the discharge liquid delivery path.

The present invention can be used for a balloon catheter system and a balloon catheter for treating arrhythmias such as atrial fibrillation, endometriosis, cancer, etc.
- 10: Balloon catheter system
- 15: Balloon catheter
- 25: Balloon
- 25a: Distal end
- 25b: Proximal end
- 30: Outer cylinder shaft
- 35: Inner cylinder shaft
- 40: Heating component
- 41: Coil electrode
- 42: Wiring
- 45: Temperature sensor
- 46: Thermosensitive part
- 47: Lead wire
- 70: Control unit
- 75: Agitator
- LD: Longitudinal direction
- LP: Liquid delivery path
- DX: Length

## Claims

1. A balloon catheter comprising:
a balloon;
an outer cylinder shaft connected to a proximal end of the balloon;
an inner cylinder shaft passing through the outer cylinder shaft to extend into the balloon to be connected to a distal end of the balloon;
a heating component disposed in the balloon for heating a liquid in the balloon; and
a temperature sensor disposed in a liquid delivery path formed between the outer cylinder shaft and the inner cylinder shaft to be in communication with the balloon.

2. The balloon catheter according to claim 1, wherein
a length along a longitudinal direction from a distal end of the outer cylinder shaft up to the temperature sensor is between 5 mm or more and 150 mm or less.

3. The balloon catheter according to claim 1 or 2, wherein:
the temperature sensor is attached to the inner cylinder shaft;
the inner cylinder shaft is relatively movable with respect to the outer cylinder shaft; and
when the inner cylinder shaft is relatively moved to a distal side with respect to the outer cylinder shaft so that the balloon is stretched, the temperature sensor is positioned in the liquid delivery path between the outer cylinder shaft and the inner cylinder shaft.

4. The balloon catheter according to claim 1 or 2, wherein
the temperature sensor is attached to the outer cylinder shaft.

5. The balloon catheter according to any one of claims 1 to 4, wherein
the temperature sensor includes a thermosensitive part, and a lead wire connected to the thermosensitive part, and
the lead wire is fixed on the inner cylinder shaft or the outer cylinder shaft, while the thermosensitive part is apart from the inner cylinder shaft and the outer cylinder shaft.

6. A balloon catheter system comprising:
a balloon catheter according to any one of claims 1 to 5; and
a control unit electrically connected to the temperature sensor and configured to determine a surface temperature of the balloon based on an output of the temperature sensor.

7. The balloon catheter system according to claim 6, comprising an agitator that repeats supply of the liquid to the liquid delivery path and discharge of the liquid from the liquid delivery path at a regular cycle, wherein
the control unit first determines a temperature fluctuation of the liquid in the liquid delivery path from an output of the temperature sensor, and then determines the surface temperature of the balloon based on the temperature fluctuation.

8. The balloon catheter system according to claim 6, comprising an agitator that repeats supply of the liquid to the liquid delivery path and discharge of the liquid from the liquid delivery path at a regular cycle, wherein
the control unit first determines a temperature fluctuation of the liquid in the liquid delivery path from an output of the temperature sensor, and then determines a local maximum value of the temperature fluctuation as the surface temperature of the balloon.

9. The balloon catheter system according to claim 6 or 7, comprising an agitator that repeats supply of the liquid to the liquid delivery path and discharge of the liquid from the liquid delivery path at a regular cycle, wherein
the control unit acquires an output from the temperature sensor at an interval less than the regular cycle.

10. The balloon catheter system according to any one of claims 6 to 9, comprising an agitator that repeats supply of a predetermined amount of the liquid to the liquid delivery path and discharge of the predetermined amount of the liquid from the liquid delivery path, wherein
a length [mm] along a longitudinal direction from the distal end of the outer cylinder shaft to the temperature sensor is a value equal to or less than a value obtained by dividing the predetermined amount [mm³] by a cross-sectional area [mm²] of the liquid delivery path.

11. The balloon catheter system according to any one of claims 6 to 10, wherein:
a wiring electrically connected to the heating component and the control unit is provided;
the temperature sensor includes a lead wire electrically connected to the control unit;
the inner cylinder shaft is relatively movable with respect to the outer cylinder shaft; and
both the wiring and the lead wire are attached to one and the same of the outer cylinder shaft and the inner cylinder shaft to extend in the liquid delivery path.

12. A balloon catheter comprising:
a balloon;
an outer cylinder shaft connected to a proximal end of the balloon;
an inner cylinder shaft passing through the outer cylinder shaft to extend into the balloon to be connected to a distal end of the balloon, the inner cylinder shaft forming between it and the outer cylinder shaft a liquid delivery path in communication with the balloon;
a coil electrode disposed in the balloon for heating a liquid in the balloon by applying a high-frequency current to the liquid during high-frequency voltage supply; and
a temperature sensor disposed at a position shielded from the high-frequency current.
